Europäisches Patentamt

European Patent Office

Office européen des brevets

(11) Numéro de publication: **0 301 961 B1**

(12) **FASCICULE DE BREVET EUROPEEN**

(45) Date de publication de fascicule du brevet: **03.11.93**

(51) Int. Cl.5: **A61K 39/002**, C12N 15/00, A61K 39/395, C12P 21/00, G01N 33/569

(21) Numéro de dépôt: **88401948.0**

(22) Date de dépôt: **27.07.88**

(54) **Antigènes d'excrétion-sécrétion spécifiques de toxoplasma gondii, leurs produits d'expression, leurs procédés d'obtention et leurs applications diagnostiques et prophylactiques.**

(30) Priorité: **31.07.87 FR 8710891**

(43) Date de publication de la demande:
**01.02.89 Bulletin 89/05**

(45) Mention de la délivrance du brevet:
**03.11.93 Bulletin 93/44**

(84) Etats contractants désignés:
**AT BE CH DE ES FR GB GR IT LI LU NL SE**

(56) Documents cités:
**EP-A- 0 082 745
FR-A- 2 564 731**

**CHEMICAL ABSTRACTS, vol. 103, no. 25, 23 décembre 1985, page 704, résumé no. 212972p, Columbus, Ohio, US; J.B. PRINCE et al.: "Cell free synthesis of toxoplasma gondii antigens", & MOL. BIOCHEM. PARASITOL. 1985, 17(2), 163-70**

**IDEM**

(73) Titulaire: **INSTITUT PASTEUR
28, rue du Docteur Roux
F-75724 Paris Cédex 15(FR)**

Titulaire: **INSTITUT PASTEUR DE LILLE
1, rue du Professeur Calmette
BP 245
F-59019 Lille Cédex(FR)**

Titulaire: **INSTITUT NATIONAL DE LA SANTE ET DE LA RECHERCHE MEDICALE (INSERM)
101, rue de Tolbiac
F-75654 Paris Cédex 13(FR)**

(72) Inventeur: **Capron, André
58, Rue du Cap. Jasmin
F-59133 Phalempin(FR)**
Inventeur: **Cesbron, Marie-France, née Delauw
46, Rue du Dr. Legay
F-59110 La Madeleine(FR)**

CHEMICAL ABSTRACTS, vol. 104, no. 21, 26 mai 1986, page 225, résumé no. 181833h, Columbus, Ohio, US; A.M. JOHNSON et al.: "Characterization and in vitro translation of toxoplasma gondii ribonucleic acid", & MOL. BIOCHEM. PARASITOL. 1986, 18(3), 313-20

CHEMICAL ABSTRACTS, vol. 105, no. 15, 13 octobre 1986, page 491, résumé no. 131792u, Columbus, Ohio, US; C. ROOUES et al.: "Preliminary immunochemical study on somatic antigens and exoantigens in toxoplasma gondii", & BULL. SOC. FR. PARASITOL. 1984, (3), 31-4

CHEMICAL ABSTRACTS, vol. 107, no. 3, 20 juillet 1987, page 459, résumé no. 21635d, Columbus, Ohio, US; C. ROOUES et al.: "Immunochemical characterization of the exo-antigenic proteins from different strains of toxoplasma gondii", & BULL. SOC. FR. PARASITOL. 1986, 4(1), 79-84

THE JOURNAL OF IMMUNOLOGY, vol. 132, no. 1, janvier 1984, pages 443-449, The American Association of Immunologists; L.H. KASPER et al.: "Identification of stage-specific sporozoite antigens of toxoplasma gondii by monoclonal antibodies"

THE JOURNAL OF IMMUNOLOGY, vol. 124, no. 6, juin 1980, pages 2578-2583, The Williams & Wilkins Co.; E. HANDMAN et al.: "Detection and characterization of membrane antigens of toxoplasma gondii"

THE JOURNAL OF IMMUNOLOGY, vol. 134, no. 5, mai 1985, pages 3426-3431, The American Association of Immunologists; L.H. KASPER et al.: "An unexpected response to vaccination with a purified major membrane tachyzoite antigen (P30) of toxoplasma gondii"

LYON MEDICAL, vol. 243, no. 12, 1980, pages 737-740; P.T. DESGEORGES et al.: "Mise en évidence et cinétique d'apparition d'exo-antigènes produits par toxoplasma gondii en culture in vitro"

Inventeur: **Darcy, Françoise**
**45, Rue Vauban**
**F-59100 Roubaix(FR)**
Inventeur: **Pierce, Raymond**
**23, Rue des Flandres**
**F-59113 Seclin(FR)**
Inventeur: **Ridel, Pierre-Richard**
**3, Allée Louis-Blanc**
**F-59290 Wasquehal(FR)**

(74) Mandataire: **Ores, Irène et al**
**CABINET ORES**
**6, Avenue de Messine**
**F-75008 Paris (FR)**

**Description**

La présente invention est relative à l'identification des antigènes excrétés-secrétés par les tachyzoïtes de Toxoplasma gondii (TG) et d'antigènes communs aux tachyzoïtes et aux bradyzoïtes de ce parasite, aux produits de traduction de ces antigènes et aux applications desdits antigènes et produits de traduction, notamment pour l'immunoprotection contre les tachyzoïtes de TG et pour le diagnostic précoce de la toxoplasmose, plus particulièrement de la toxoplasmose congénitale.

Le protozoaire parasite Toxoplasma gondii, membre de la classe des Sporozoa et de l'ordre des Coccidia est un parasite intracellulaire qui se reproduit dans une grande variété de types de cellules à l'intérieur de ses hôtes, qui sont des mammifères. Chez l'être humain, l'atteinte la plus significative causée par cet organisme a pour siège le foetus en développement et les patients présentant un déficit immunitaire (SIDA, etc...). La toxoplasmose est responsable d'atteintes oculaires et cérébrales sévères et même mortelles chez les nouveau-nés contaminés par voie transplacentaire, ainsi que chez les patients immunodéficients.

Chez l'homme, deux formes du parasite ont été décrites : le "tachyzoïte", qui est la forme multiplicative rencontrée au cours de la phase aiguë de la maladie, et le "bradyzoïte", forme résistante qui persiste enkystée dans les tissus nerveux et est vraisemblablement responsable de l'entretien d'une immunité durable à la réinfection.

Ce parasite est un pathogène important, non seulement chez l'homme mais également en médecine vétérinaire et il est très largement répandu géographiquement, à telle enseigne qu'on a pu établir que près de 500 millions de personnes dans le monde entier présenteraient une réaction sérologique positive à l'infection.

HUGHES, "CURRENT TOPICS IN MICROBIOLOGY AND IMMUNOLOGY", Vol. 120 (1985), SPRINGER Ed., pages 105-139, passe en revue les différents tests de sérodiagnostic disponibles : test de coloration de SABIN et FELDMAN, standardisé par BEVERLY et BEATTLE (1958) et perfectionné par FELDMAN et LAMB (1966), WALDELAND (1976) et BALFOUR et Al. (1982), qui requiert des parasites vivants, de grandes quantités de sérum humain normal et des spécialistes bien entraînés pour sa réalisation ; test de détection d'anticorps par immunofluorescence, qui présente les mêmes inconvénients que le test de coloration et qui a été mis au point par REMINGTON (1968) et perfectionné par KARIM et LUDLAM (1975) ; les tests d'hémagglutination, dont les résultats sont trop tardifs, notamment pour la détermination de la présence du parasite chez la femme enceinte ; test ELISA pour la détection d'anticorps de Toxoplasma, par isolement d'IgM in situ sur la microplaque de test (WIELARRD et Al., 1983). Cependant, dans les test sérologiques utilisant des antigènes solubles, ces derniers sont obtenus par simple extraction (lyse à l'eau, congélation et décongélation ou éclatement par ultra-sons de tachyzoïtes de Toxoplasma). Ces techniques n'assurent la présence d'antigènes de membrane dans le système de test que s'ils sont aisément solubles dans l'eau ou le tampon ou si l'étape finale de centrifugation ne laisse subsister que peu de fragments de membrane en suspension dans la solution.

Les différents tests mis en oeuvre reposent sur la formation d'anticorps pour la détection de différents antigènes, d'où l'importance de l'identification desdits antigènes dans le but d'améliorer la fiabilité et la sensibilité des tests. Trois antigènes de Toxoplasma avaient pu être identifiés par CHORDI et Al. (J. IMMUNOL., 93 (1964) pages 1034-1044), puis confirmés par HUGHES et BALFOUR (PARASITE IMMUNOL., 3 , (1981), pages 235-248) et leur origine intracellulaire établie. L'analyse de polypeptides de Toxoplasma, provenant d'organismes traités par ultra-sons, par SDS - PAGE et par fixation de concanavaline marquée à l'$^{125}$I, a montré l'existence de 9 polypeptides intracellulaires d'origine parasitaire, de poids moléculaires compris entre 20 000 et 98 000, dont aucun n'est glycosylé [JOHNSON AM., McDONALD PJ. et NEOH SH. (1981), Biochem. Biophys. Res. Commun., 100, 934]. Il a été montré ultérieurement que 10 polypeptides obtenus par sonication, puis précipitation par de l'immun-sérum de souris, présentaient des propriétés antigéniques.

Des exo-antigènes obtenus à partir de cultures cellulaires de TG ont également été décrits [CHEMICAL ABSTRACTS, vol. 105, n° 15, 13 octobre 1986, p. 491, résumé n° 131792u ; BULL. SOC. FR. PARASITOL., 1986, 4(1), 79-84].

La technique de KOHLER et MILSTEIN d'hybridation avec des cellules lymphoïdes a permis de développer des anticorps monoclonaux pour sonder la structure antigènique de Toxoplasma. HUGHES [ (1985) loc. cité ] mentionne qu'à la date de sa Publication 32 anticorps monoclonaux avaient été décrits et réagissent contre des antigènes de membrane de Toxoplasma. Tous ont été définis du point de vue sérologique ; 4 présentent une réactivité à la fois contre les composants de la membrane et du cytoplasme et 28 sont positifs uniquement dans des tests de reconnaissance des antigènes de membrane. Les tests d'immunoprécipitation et de SDS - PAGE appliqués à ces anticorps monoclonaux ont montré que la majorité d'entre eux réagissent contre des antigènes de poids moléculaires 35KD, 27KD et 14KD. Les anticorps résultant des clones 3E6 et 2G11 précipitent tous deux les antigènes de P.M. 35KD et 14KD [HANDMAN et AL. J. IMMUNOL., (1980) pages 2578-2583], de même que les anticorps monoclonaux FMC 18, FMC 22, FMC 23 développés par JOHNSON et Al. [J. EXP. BIOL. MED. SCI, 59 (1981), pages 303-306], tous ces anticorps étant spécifiques aux membranes des tachyzoïtes, KASPER et AL. [J. IMMUNOL. (1982), 129, pages 1694-1699 et (1983) 130, pages 2407-2412] ont récemment décrit deux antigènes précipitables de poids moléculaires 22KD (P22) et 30KD (P30).

De plus, JOHNSON et Al. [J. PROTOZOOL (1983), 30, pages 351-356] ont montré que des anticorps monoclonaux qui réagissent contre le même antigène de membrane peuvent reconnaître différents épitopes et HUGHES suggère en relatant ces derniers travaux (Page 11, loc. cité) que des études similaires pourraient être effectuées sur d'autres anticorps monoclonaux qui reconnaissent un antigène commun et pourraient permettre de définir la structure moléculaire des épitopes concernés.

Dans sa revue apparemment exhaustive (1985, loc. cité), HUGHES souligne également l'importance de la détection des antigènes circulants dans les fluides corporels : alors que l'infection par Toxoplasma gondii conduit normalement à une réponse humorale rapide démontrable par des tests sérologiques tels que ceux énumérés plus haut, ceux-ci peuvent ne pas être suffisants pour le clinicien lorsqu'il faut diagnostiquer rapidement et avec précision la présence d'une infection aiguë à partir d'un échantillon unique de sérum . Ceci est particulièrement important dans le cas d'un patient immunodéficient qui représente un danger de dissémination ou en cas d'hypogammaglobulinémie sous-jacente.Si la détection d'une antigénémie permet de confirmer dans ces cas comme pendant la phase aiguë de l'infection, la présence d'une infection active, dans la toxoplasmose congénitale, la détection d'antigènes dans des fluides corporels tels que le liquide amniotique ou le liquide céphalorachidien, peut également fournir une indication au sujet de la gravité de l'infection par Toxoplasma et permettre, par conséquent, de prendre des décisions sur le plan de la thérapeutique et du pronostic à adopter.

Des études de double diffusion ont montré qu'un antigène circulant détecté dans du sérum de souris atteintes de toxoplasmose aiguë, présente des composants communs avec des extraits de Toxoplasma lysés par de l'eau, est une protéine thermolabile (56°C/30 minutes) et son poids moléculaire (obtenu après l'avoir isolé par chromatographie d'affinité) est supérieur à 100 000, de même que des antigènes circulants présents dans du sérum de rats infectés.

Les antigènes circulants peuvent être libérés de deux façons : soit par secrétion active par le parasite, soit par immunolyse. Il a été montré que les antigènes circulants ne présentent pas d'identité immunologique avec de simples extraits aqueux ou des lavages péritonéaux, ce qui implique que l'antigénémie n'est pas simplement un résultat de parasites mourants ou de la libération d'antigènes de cellules infectées. Toutefois l'antigène présent dans les immuncomplexes circulants récemment décrits par SIEGEL et REMINGTON [CLIN. EXP. IMMUNOL. (1983) 52, pages 157-163] n'a pas encore été identifié.

Par ailleurs JOHNSON [PATHOLOGY (1985), 17, pages 9-19] souligne que la plupart des travaux sur le sujet ont utilisé la forme "tachyzoïte" du parasite comme source d'antigène, alors que la voie naturelle de transmission de la toxoplasmose est l'ingestion orale soit des sporozoïtes présents dans les oocystes (qui ne se trouvent que chez le chat), soit des bradyzoïtes dans les kystes des tissus. Il a été démontré qu'il existe un antigène commun aux 5 formes (schizonte, gamétocyte, sporozoïte, tachyzoïte, bradyzoïte) du cycle vital de Toxoplasma et qu'un antisérum marqué à la fluorescéine, produit contre le tachyzoïte, réagit spécifiquement avec les 4 autres formes [CARVER RK., GOLDMAN M., Am. J. Clin. Pathol. (1959), 32, 159-64 ; DUBEY JP., MILLER NL., FRENKEL JK., J. Exp. Med. (1970), 132, 636-62 ; FRENKEL JK., DUBEY JP., MILLER NL., Science (1970), 167, 893-6]. Or, d'autres Auteurs [LUNDE et JACOBS, J. PARASITOL. (1983) 69, 806-808] ont montré qu'il y a des différences antigéniques entre les tachyzoïtes et les bradyzoïtes : de l'antisérum anti-bradyzoïtes, marqué à la fluorescéine, ne réagit que contre les bradyzoïtes, alors que l'anti-sérum anti-tachyzoïtes réagit aussi bien contre les tachyzoïtes que contre les bradyzoïtes. De même, les essais effectués par KASPER et Al. [J. IMMUNOL. (1984), 132, pages 443-449] ont corroboré ce résultat, encore que les différences antigéniques qu'ils ont montrées entre les sporozoïtes et les tachyzoïtes puissent permettre d'envisager la possibilité du développement de tests de diagnostic aptes à différencier les deux voies d'infection par Toxoplasma.

Selon HUGHES (loc. cité), la vaccination contre la toxoplasmose ne pourra être envisagée avec les anticorps monoclonaux, avec plus de succès que les tentatives antérieures (vaccins utilisant des organismes tués ou des organismes atténués) que dans la mesure où il pourra être établi que les antigènes isolés par chromatographie d'affinité en utilisant les anticorps FMC19 et FMC22 isolés par JOHNSON et Al. (loc. cité), sont protecteurs et qu'ils sont aptes à induire une réponse humorale. En effet, les résultats rapportés jusqu'alors ne sont pas suffisamment probants à ce sujet, à ce jour : JOHNSON et Al. (1983, loc. cité) ont trouvé que les anticorps monoclonaux FMC19 et FMC22 peuvent induire une protection contre des souches virulentes et très virulentes de Toxoplasma, en réagissant contre les antigènes de PM 35KD et 14KD ; cependant, d'autres anticorps monoclonaux qui précipitent les mêmes antigènes, à l'analyse par SDS - PAGE, ne procurent pas le même degré de protection, en sorte que les Auteurs ont supposé qu'ils reconnaissaient des épitopes différents sur le même antigène. Les deux anticorps monoclonaux qui induisent une protection sont des isotypes IgG3 et IgG1 respectivement. Les deux anticorps monoclonaux qui n'induisent pas de protection significative sont respectivement des isotypes IgGl (FMC23) et IgG2a (2G11). Si, en ce qui concerne FMC23, l'absence de protection serait effectivement à rapporter à la différence entre les épitopes reconnus par rapport à FMC 19 ou FMC 22, en ce qui concerne 2G11 elle pourrait également être due aux particularités différentes de la sous-classe IgG2a par rapport aux sous-classes IgG1 et IgG3.

KASPER et Al. [J. IMMUNOL. (1985) 134, pages 3426-3431] ont décrit des tentatives d'immunisation de souris par une protéine de membrane de tachyzoïtes de Toxoplasma gondii purifiée, la protéine P 30, et un anticorps monoclonal dirigé contre cet antigène ; ils ont observé chez les souris traitées une augmentation statistiquement significative de la mortalité par rapport à des souris témoins non-immunisées et, chez les souris vaccinées, un nombre accru de kystes tissulaires intercérébraux par rapport à un groupe témoin. Des résultats similaires ont été obtenus avec une immunisation passive par transfert, à l'aide d'anticorps monoclonaux dirigés contre l'antigène P 30. Comme les bradyzoïtes ne contiennent ni l'antigène P30, ni l'antigène P22, il n'est pas exclu, selon les Auteurs, que cet échec soit dû à la résistance des parasites aux anticorps anti-tachyzoïtes.

Les travaux les plus récents dans ce domaine ont fait l'objet d'Abstracts publiés dans la Revue "MOLECULAR STRATEGIES OF PARASITIC INVASION" éditée par l' UCLA en Janvier 1986 :

- BOOTHROYD, BURG, NAGEL, OSSORIO, PERELMAN, KASPER, WARE, PRINCE, SHARMA et REMINGTON (Abstract C19) ont utilisé les techniques mettant en oeuvre l'ADN recombinant et les anticorps monoclonaux dans le but de mieux comprendre les propriétés biologiques et les propriétés inductrices de la toxoplasmose ; à cet effet, ils ont utilisé les anticorps monoclonaux et polyclonaux dirigés contre les principaux antigènes de surface du parasite, pour isoler et caractériser leurs gènes respectifs. Ils ont produit des séquences partielles ou complètes de nucléotides de certains de ces gènes et ils ont aussi cloné et partiellement séquencé les gènes d'$\alpha$- et de $\beta$-tubulines qui ne sont présents chacun qu'en un seul exemplaire dans Toxoplasma gondii. Ces derniers ont fait l'objet d'une caractérisation plus poussée rapportée dans l'Abstract C 59 par NAGEL et BOOTHROYD.

- BURG, PERELMAN et BOOTHROYD (Abstract C 85) décrivent l'élaboration d'une banque d'expression dans le phage $\lambda$Gt11 en utilisant l'ADN génomique de Toxoplasma gondii et l'isolement à partir de cette banque, à l'aide d'immun-sérums de souris infectées par Toxoplasma gondii, d'une séquence possédant un potentiel codant pour un déterminant antigénique majeur du parasite, qui est le gène TgB1. Cette séquence semble spécifique à Toxoplasma gondii et le déterminant antigénique produit dans le clone TgB1 $\lambda$gt11 est reconnu par de l'immun-sérum de souris, de rat et d'homme.

Ces Auteurs décrivent également le criblage d'une banque d'expression de l'ADNc du Toxoplasma - (également construite à l'aide du phage $\lambda$ gt11) pour isoler des séquences codant pour les antigènes présents sur la surface du parasite, à l'aide d'antisérum polyclonal dirigé contre les protéines antigéniques de surface P 30 et P 22 purifiées. En utilisant l' ADNc de l'antigène P 22 comme sonde dans un immunotransfert de Northern, ils ont détecté une seule espèce d'ARN comprenant environ 1600 nucléotides.

- PRINCE, REMINGTON et SHARMA (Abstract C 106) ont caractérisé un antigène de Toxoplasma gondii reconnu par un anticorps monoclonal (mab)$F_3G_3$ dont ils ont déterminé qu'il procure à des souris une immunité protectrice de longue durée (supérieure à 9 mois). Le clonage des gènes codant pour les antigènes de Toxoplasma gondii a été obtenu en élaborant une banque d' ADNc de tachyzoïtes dans le vecteur d'expression gt11, puis criblage de celle-ci à l'aide d'un anticorps polyclonal de souris dirigé contre l'antigène $F_3G_3$.

Des clones recombinants isolés par criblage à l'aide de la sonde anticorps monospécifique, contiennent un insert d' ADN de Toxoplasma gondii qui code pour 1/6e de la protéine intacte et l'antigène recombinant qui résulte de ces clones est également reconnu par (mab)$F_3G_3$.

- PRINCE, KOVEN-QUINN, REMINGTON et SHARMA (Chemical Abstract, vol. 103, n° 25, 23 décembre 1985, p. 704, résumé n° 212972p) ont décrit l'isolement d'ARNm polyadénylés de tachyzoïtes de la souche RH de Toxoplasma gondii et leur traduction in vitro dans des systèmes de traduction constitués par un extrait de germes de blé. La traduction des ARNm dans ledit système nécessite la présence de spermine et de ARNt exogènes.

Les travaux effectués dans le domaine de la Toxoplasmose ont largement démontré : - la nécessité d'identifier des antigènes du stade tachyzoïte et du stade bradyzoïte présentant des épitopes communs, pour permettre l'établissement d'une immunité concomitante ; - la nécessité de l'identification des antigènes excrétés-sécrétés par les tachyzoïtes et de l'évaluation de l'importance de leur rôle dans l'immunité protectrice - et la nécessité de la mise en évidence du rôle des anticorps de classe IgE dans l'immunité protectrice contre la toxoplasmose.

La présente invention a pour objet une préparation antigénique de *Toxoplasma gondii* (TG), caractérisée

\* en ce qu'elle est susceptible d'être obtenue par :
- incubation d'une suspension de tachyzoïtes de *Toxoplasma gondii* pendant environ 3 heures dans un milieu acellulaire de type RPMI 1640 contenant 10 % de sérum décomplémenté d'origine humaine ou animale ;
- centrifugation de la suspension incubée et récupération des surnageants dépourvus de corps parasitaires et contenant les antigènes ES ;
- filtration desdits surnageants, pour l'obtention de ladite préparation antigénique ; et éventuellement
- concentration et congélation à - 70 ° C de ladite préparation antigénique, et

\* en ce qu'elle contient un mélange d'antigènes d'excrétion-sécrétion (antigènes ES) de tachyzoïtes de TG, capables d'induire une réponse humorale protectrice de type IgE contre la toxoplasmose.

Dans le cadre de leurs recherches dans le domaine des maladies parasitaires humaines et plus particulièrement de la toxoplasmose, les Inventeurs sont parvenus à identifier les mécanismes effecteurs et immunorégulateurs de ces affections et ils ont notamment pu établir l'importance des antigènes excrétés-sécrétés dans l'élaboration de la réponse immunoprotectrice, alors que les antigènes de membrane sont plus particulièrement impliqués dans l'induction d'isotypes aussi bien effecteurs que bloquants.

Ces découvertes ont conduit les Inventeurs à chercher à isoler lesdits antigènes excrétés-sécrétés, dans le but, notamment, de la mise au point d'un vaccin contre la toxoplasmose.

Conformément à l'invention, la préparation antigénique est constituée par des antigènes d'excrétion-sécrétion de tachyzoïtes de TG, de poids moléculaires respectifs 185 kDa, 170 kDa, 155 kDa, 105 kDa, 98 kDa, 84 kDa, 68 kDa, 57 kDa, 53 kDa, 45 kDa, 43 kDa, 39 kDa, 35 kDa, 34 kDa, 31 kDa, 30 kDa, 26 kDa, 25 kDa, 24 kDa et 20 kDa.

Conformément à l'invention, les antigènes ES de *Toxoplasma gondii* sont susceptibles d'être obtenus à partir de la préparation antigénique conforme à l'invention par :
- immunoprécipitation des différents antigènes ES contenus dans ladite préparation, préalablement radio-marqués, par mise en contact de cette dernière avec un sérum de mammifère, notamment sérum humain ou animal convenable choisi dans le groupe qui comprend les sérums prélevés chez des malades et reconnus comme séropositifs (soit de phase aiguё contenant des IgM spécifiques, soit de phase subaigüe contenant à la fois des IgM et des IgG, soit de phase chronique contenant des IgG) et les sérums obtenus par immunisation par une préparation antigénique conforme à l'invention et contenant des anticorps anti-ES ;
- piégeage des complexes antigènes-anticorps formés (immunoprécipités) sur des billes de protéine A-Sépharose, éventuellement traitées de manière à fixer les anticorps de type IgG et les anticorps de type IgM ;
- séparation desdits antigènes ES par centrifugation et récupération des surnageants contenant les immunoprécipités dissociés, et éventuellement
- électrophorèse des immunoprécipités en gels de polyacrylamide.

Selon une disposition avantageuse de l'invention, les antigènes de poids moléculaire 185 kDa, 170 kDa, 155 kDa, 105 kDa, 57 kDa, 53 kDa, 45 kDa, 43 kDa, 39 kDa, 35 kDa, 34 kDa, 31 kDa, 30 kDa, 26 kDa, 25 kDa et 24 kDa sont reconnus au cours de la phase chronique.

Selon une autre disposition avantageuse de l'invention, l'antigène ES de poids moléculaire 105 kDa correspond à un marqueur de la phase chronique de la toxoplasmose.

Selon encore une autre disposition avantageuse de l'invention, l'antigène ES de poids moléculaire 84 kDa correspond à un marqueur transitoire de la phase chronique de la toxoplasmose.

Selon une autre dispostion avantageuse de l'invention, l'antigène ES de poids moléculaire 98 kDa correspond à un marqueur de la phase aiguё de la toxoplasmose.

Selon une autre disposition avantageuse de l'invention, l'antigène ES de poids moléculaire 68 kDa correspond à un marqueur de la phase aiguё de la toxoplasmose.

Egalement conformément à l'invention, les antigènes ES sont susceptibles d'être obtenus à la fois par immunoprécipitation de la préparation antigénique conforme à l'invention :
- avec un sérum convenable choisi dans le groupe qui comprend les sérums prélevés chez des malades et les serums obtenus par immunisation par une préparation antigénique conforme à l'invention et contenant des anticorps anti-ES et

● avec des sérums obtenus par immunisation par des antigènes de bradyzoïtes,

en ce qu'ils correspondent à des antigènes ES communs aux tachyzoïtes et aux bradyzoïtes de TG, soit de poids moléculaire d'environ 25 kDa-24 kDa, soit de poids moléculaire d'environ 43 kDa et de nature glycoprotéique.

Ladite immunoprécipitation est avantageusement suivie d'une dissociation des immunoprécipités formés, d'une électrophorèse en gel de polyacrylamide (SDS-PAGE), d'une autoradiographie des gels et de leur révélation.

Les antigènes de bradyzoïtes d*e Toxoplasma gondii* sont isolés de kystes récupérés de broyats de tissus de mammifères, tels que souris notamment, infectés par la forme chronique de *Toxoplasma gondii,* lesquels kystes sont purifiés sur un gradient approprié, soniqués puis broyés à l'état congelé et centrifugés pour recueillir un surnageant contenant les antigènes cytoplasmiques (fraction $S_1$) et le culot dont on libère les antigènes membranaires (fraction $S_2$) à l'aide d'un détergent.

La présente invention a également pour objet un produit de traduction d'ARNm de tachyzoïtes, caractérisé en ce qu'il comprend au moins un antigène ES conforme à l'invention et en ce qu'il est reconnu par des sérums humains de phase chronique ou de phase aiguë de l'infection à *Toxoplasma gondii.*

Selon une disposition avantageuse de l'invention, le produit de traduction d'ARNm de tachyzoïtes tel que défini ci-dessus, est susceptible d'être obtenu par traduction *in vitro* des ARNm, extraits de lysats de tachyzoïtes, dans du lysat de réticulocytes, en présence d'un mélange d'aminoacides dépourvus de L-méthionine et comprenant la $^{35}$S-méthionine, en milieu tamponné.

La présente invention a également pour objet un procédé de clonage des produits de traduction conformes à l'invention, caractérisé en ce qu'il comprend :

- l'extraction d'ARNm à partir de lysats de tachyzoïtes,
- et la synthèse d'ADNc à partir desdits ARNm de tachyzoïtes précédemment obtenu, par action de la transcriptase réverse d'AMV, la banque d'ADNc ainsi formée étant ensuite criblée pour identifier les phages recombinants dont l'insertion d'ADNc dirige la synthèse d'une protéine ou fraction de protéine portant les épitopes reconnus par les anticorps anti-ES et y sélectionner les clones ES reconnus par des sérums humains, les clones retenus étant caractérisés par sélection d'anticorps.

La présente invention a également pour objet un sérum riche en anticorps IgE propres à conférer une protection significative contre la toxoplasmose et en particulier contre les tachyzoïtes, caractérisé en ce qu'il est susceptible d'être obtenu à partir d'animaux immunisés par des préparations antigéniques, des antigènes ES ou des produits de traduction d'ARNm de tachyzoïtes conformes à l'invention.

La présente invention a également pour objet un anticorps monoclonal, caractérisé en ce qu'il est susceptible d'être produit par des hybridomes résultant de la fusion de cellules d'animaux immunisés par les antigènes ES conformes à l'invention avec des cellules tumorales.

La présente invention a, de plus, pour objet un vaccin propre à procurer une protection significative contre la toxoplasmose, caractérisé en ce qu'il contient, en tant que constituant actif, une préparation antigénique ou un antigène ES conforme à l'invention, aptes à induire une réponse de type IgE.

Les antigènes ES conformes à l'invention trouvent également application dans le traitement de la toxoplasmose.

La présente invention a également pour objet des produits de diagnostic de la toxoplasmose, y compris de la toxoplasmose congénitale, caractérisés en ce qu'ils contiennent en tant que constituants actifs, une préparation antigénique ou des antigènes ES conformes à l'invention.

Selon une disposition avantageuse de l'invention, les produits de diagnostic sont caractérisés en ce qu'ils comprennent l'antigène de poids moléculaire 105 kDa et en ce que qu'ils correspondent à un marqueur de la phase chronique de la toxoplasmose.

Selon une autre disposition avantageuse de l'invention, les produits de diagnostic sont caractérisés en ce qu'ils comprennent l'antigène de poids moléculaire 84 kDa, qui correspond à un marqueur transitoire de la phase chronique de la toxoplasmose.

Selon encore une autre disposition avantageuse de l'invention, les produits de diagnostic sont caractérisés en ce qu'ils comprennent l'antigène de poids moléculaire 98 kDa, qui correspond à un marqueur de la phase aiguë de la toxoplasmose.

Selon une autre disposition avantageuse de l'invention, les produits de diagnostic sont caractérisés en ce qu'ils comprennent l'antigène de poids moléculaire 68 kDa, qui correspond à un marqueur de la phase aiguë.

Selon encore une autre disposition avantageuse de l'invention, les produits de diagnostic sont caractérisés en ce qu'ils comprennent l'antigène de poids moléculaire 24 kDa, qui correspond à un marqueur tardif de la phase chronique de la toxoplasmose ainsi qu'à un marqueur d'immunité protectrice.

La présente invention a, en outre, pour objet un procédé de détection et/ou de dépistage de la toxoplasmose, caractérisé en ce qu'il comprend la mise en contact d'un sérum humain avec un produit de diagnosic conforme à l'invention.

Outre les dispositions qui précèdent, l'invention comprend encore d'autres dispositions qui ressortiront de la description qui va suivre.

L'invention sera mieux comprise à l'aide du complément de description qui va suivre, qui se réfère à des exemples de préparation d'antigènes de Toxoplasma gondii, et plus particulièrement d'antigènes ES, de caractérisation de ceux-ci et de préparation d'anticorps anti-ES.

Il doit être bien entendu, toutefois, que ces exemples sont donnés uniquement à titre d'illustration de l'objet de l'invention, dont ils ne constituent en aucune manière une limitation.

### EXEMPLE 1 - Préparation d'antigènes d'excrétion-sécrétion des tachyzoïtes (antigènes ES)

a) Conditions d'excrétion :

La viabilité des parasites est évaluée par un test préalable de survie à l'érythrosine B : 30 microlitres de la suspension de tachyzoïtes et 30 microlitres d'une solution d'érythrosine B à 4 milligrammes par millilitre en tampon PBS 0,01 M pH 7,2 sont mis en contact sur un bain de glace pendant 5 minutes exactement avant observation microscopique. On détermine alors le pourcentage de tachyzoïtes vivants, respectés par le colorant et apparaissant réfringents, et de tachyzoïtes morts, pénétrés par l'érythrosine et colorés en rose.

Ce test de survie a permis d'établir que les conditions de survie optimales correspondent à une excrétion active, sans libération d'antigènes cytoplasmiques par lyse des parasites.

Les antigènes ES sont obtenus en incubant pendant 3 heures les tachyzoïtes (à raison de $1,8 \times 10^8$ tachyzoïtes par tube de 1,5 millilitre de milieu d'excrétion) dans du milieu RPMI 1640 contenant 10 % de sérum décomplémenté d'origine humaine ou animale.

La comparaison de la cinétique d'excrétion en présence de sérums de diverses origines a montré que les meilleurs résultats sont obtenus par ordre décroissant avec des sérums humains, de veau foetal, de lapin, de rat et de souris. Les antigènes excrétés en présence de ces différents sérums sont qualitativement identiques.

A la fin des 3 heures d'incubation, une centrifugation à 2800 tours par minute pendant 10 minutes permet de séparer les surnageants contenant les antigènes ES. Ces surnageants sont ensuite filtrés sur membrane Millipore® (0,22 $\mu$m) puis concentrés 10 fois par centrifugation à 5000 tours par minute pendant 40 minutes en tubes Centricon 10® (Amicon). Ces préparations d'antigènes ES sont conservées par congélation à - 70°C après addition d'une solution d'inhibiteurs de protéases présentant la composition suivante :
- pour 100 ml de PBS 10 mM pH 7,2 =
- Fluorure de Phénylméthylsulfonyl (PMSF) (Sigma)®: 100 $\mu$l d'une solution à 40 mM dans l'éthanol absolu
- N p Tosyl-L-lysine-chlorométhylcétone (TLCK), (Sigma®) = 7,3 mg
- Acide Ethylène diamine tétraacétique, sel disodique (EDTA) (Prolabo)® = 75 mg
- N Tosyl-L-Phénylalanine-chlorométhylcétone (TPCK) (Sigma)® = 7 mg

b) Marquage métabolique par la méthionine $^{35}$S :

Après filtration sur membrane Nuclepore® (3$\mu$m) et lavages en milieu RPMI, les tachyzoïtes sont mis en suspension, à raison de $1,2.10^8$ tachyzoïtes/ml dans du milieu RPMI 1640 sans méthionine ni cystine, contenant 10 % de sérum décomplémenté par chauffage à 56°C pendant 30 minutes : ce sérum a été préalablement dialysé une nuit à +4°C contre une solution de NaCl 9 $^0$/oo et filtré sur membrane Millipore® (0,22 $\mu$m).

La suspension de tachyzoïtes est répartie sous un volume de 1,5 millilitre dans des tubes à hémolyse placés à 37°C et soumis pendant 30 minutes à une agitation douce.

On ajoute ensuite au milieu de cette culture la méthionine marquée (225 microcuries par tube) et on réincube à 37°C sous agitation douce. Au bout d'une heure, on ajoute dans chaque tube 15 microlitres de méthionine et 15 microlitres de cystine froide du Kit RPMI (Difco)® avant de replacer les tubes à 37°C sous agitation pendant 1 h.30. Après centrifugation à 2800 tours par minute, pendant 10 minutes, on sépare pour les traiter différemment les surnageants contenant les antigènes ES, des culots constitués des parasites entiers.

Après filtration sur membrane Millipore® (0,22$\mu$m), puis passage sur colonnes de Sephadex® G25 M (PD 10 de Pharmacia) pour éliminer la méthionine libre, les surnageants sont concentrés 10 fois par centrifugation à 5000 tours par minutes, pendant 40 minutes, en tubes Centricon 10® (Amicon®). Les antigènes ES sont conservés par congélation à -70°C après addition d'inhibiteurs de protéases tels que décrits plus haut.

c) Marquage des antigènes contenus dans le culot, par la méthionine $^{35}$S :

Les culots obtenus ci-dessus sont lavés deux fois en PBS 0,01 M pH 7,2 et repris par de l'eau distillée avant d'être soumis à 6 cycles de congélation (en azote liquide) et décongélation (en bain-marie à 37°C) successifs.

Une nouvelle centrifugation (2800 tours par minute pendant 10 minutes) permet de séparer les surnageants (contenant les antigènes cytoplasmiques), des culots, à partir desquels seront extraits les antigènes membranaires.

L'extraction et la solubilisation des antigènes membranaires est obtenue par traitement au Nonidet P 40 ou au CHAPS( /(3 cholamido-propyl) diméthylammonio/-propanesulfonate) (FLUKA), suivi d'une centrifugation à 5000 tours/mn pendant 30 minutes : les antigènes membranaires solubilisés sont contenus dans le surnageant. Les antigènes cytoplasmiques et membranaires

sont conservés par congélation à -70°C en présence d'une solution d'inhibiteurs de protéases.

d) Marquage des antigènes membranaires des tachyzoïtes par $^{125}$I (Iodogen) :

La méthode de marquage utilisée est une modification de la méthode utilisant l'IODO-GEN décrite par HOWARD et Al. [HOWARD, R.J., KAVSHAL, D.C., CARTER, R., J. PROTOZOOL., (1982) 22, 114]. Les tachyzoïtes obtenus par lavages péritonéaux de souris infectées par la souche R.H. de T. gondii sont lavés deux fois par du PBS 0.01 M pH 7,2 après

filtration sur filtres Nuclépore 3μm. On dissout 1 milligramme d'IODO-GEN (PIERCE Chemical co., Rockford, IL) dans 10 millilitres de chloroforme et on transfère 625 microlitres de cette solution dans un tube en verre. On évapore alors le solvant sous courant d'azote et on ajoute $10^8$ tachyzoïtes. Après 10 minutes de contact à 4°C avec 500 μc d'$^{125}$I, on transfère le mélange dans un autre tube contenant du PBS 0,1 M, pH 7,2, NaI 10 mM pour arrêter la réaction. On lave ensuite trois fois les tachyzoïtes en PBS 0,1 M, pH 7,2. Les antigènes membranaires sont ensuite extraits par le Nonidet P40 ou le CHAPS

## EXEMPLE 2 - Préparation d'antigènes des bradyzoïtes :

a) Production de bradyzoïtes ; extraction des antigènes :

Les bradyzoïtes, stade enkysté du toxoplasme, sont produits en infectant par voie intrapéritonéale des souris SWISS avec environ 30 kystes de la souche "chronique" 76 K de Toxoplasma gondii. Après 4 à 6 semaines, les souris infectées présentent de 1000 à 2000 kystes intracérébraux.

Les kystes, récupérés à partir de broyats de cerveaux de souris infectées, ont été tout d'abord purifiés sur gradient de gomme arabique, selon la technique de Nakabayashi et Motomura (1968). La technique d'extraction des antigènes des bradyzoïtes (présents dans les kystes) choisie est la suivante : les kystes sont soumis aux ultra-sons (quatre fois 1 minute), puis broyés à l'état congelé par 17 passages à la X-Presse. Après centrifugation à 32000 g pendant 2 heures à +4°C, le surnageant contenant les antigènes cytoplasmiques (fraction $S_1$) est recueilli et le culot est traité par le détergent CHAPS qui libère les antigènes membranaires (fraction $S_2$). Les différentes étapes sont effectuées en présence d'une solution d'inhibiteurs de protéases.

b) Marquage des antigènes des bradyzoïtes par $^{125}$I :

Le marquage est réalisé suivant la technique de HUNTER & GREENWOOD (chloramine T) [(1962) Nature, London, 194, 495-496].

Les fractions $S_1$ et $S_2$ (contenant de 250 à 500 μg de protéines) sont placées dans un faible volume de tampon phosphate de sodium 0,5 M final, pH 7,4 (300 à 400 μl).

La réaction de marquage est identique dans les deux cas :
- addition de 500 μCi Na$^{125}$I (Amersham) + 100 μl d'une solution à 2 mg/ml de chloramine T (Merck)
- incubation 1 min. 30 à température ambiante sous agitation douce
- addition de 100 μl d'une solution à 4 mg/ml de métabisulfite de sodium (Prolabo)®
- dialyse sur une colonne PD10 (Pnarmacia) contre du PBS pour éliminer l'Iode libre.

## EXEMPLE 3 - Isolement par immunoprécipitation et électrophorèse SDS-PAGE, des antigènes ES :

a) Immunisation par les antigènes d'excrétion-secrétion des tachyzoïtes :
- Rats Fischer

Immunisation par 4 injections sous-cutanées réalisées avec des intervalles de 10 à 14 jours.

Produit injecté : antigènes ES obtenus à l'Exemple 1 correspondant à l'excrétion de $10^8$ tachyzoïtes sous un volume de 300 $\mu$l, additionnés de 300 $\mu$l d'adjuvant incomplet de Freund.

- Souris Balb/C

Immunisation par 4 injections sous-cutanées réalisées à 10 jours d'intervalle.

Produit injecté : antigènes ES obtenus à l'Exemple 1 correspondant à l'excrétion de 0,5 x $10^8$ tachyzoïtes sous un volume de 100 $\mu$l, additionnés de 100 $\mu$l d'adjuvant incomplet de Freund.

- Lapins

Les lapins ont été immunisés selon la technique de Vaitukaitis et Al. [J. Clin. Endocr.(1971), 33, 988-991]: 40 injections intradermiques sont réalisées avec 1 ml d'antigènes ES (correspondant à l'excrétion de $8.10^8$ tachyzoïtes additionnés d'1 ml d'adjuvant complet de Freund) + 1 injection intra-musculaire de 1 ml de vaccin anti-coquelucheux (Vaxicoq®, Institut Mérieux). Des rappels avec 1/10e de la dose de départ sont réalisés toutes les trois semaines à partir de 1 mois par voie intra-musculaire.

b) Immunisation par les antigènes des bradyzoïtes :

- Rats Fischer

4 injections sous-cutanées avec des intervalles de 10 à 14 jours.

- Antigènes solubles (S1)

Par injection : environ 60 $\mu$g d'antigènes solubles (S1) obtenus à l'Exemple 2 dans 300 $\mu$l de PBS 10mM pH 7,2 + 300 $\mu$l d'adjuvant incomplet de Freund.

- Antigènes membranaires (S2)

Par injection : 130 $\mu$g d'antigènes membranaires (obtenus à l'Exemple 2) dans 300 $\mu$l d'adjuvant incomplet de Freund.

- Souris Balb/C

4 injections sous-cutanées à 10 jours d'intervalle.

- Antigènes solubles (S1)

Par injection : 15 $\mu$g d'antigènes (obtenus à l'Exemple 2) dans 75 $\mu$l de PBS 10 mM pH 7,2 + 75 $\mu$l d'adjuvant incomplet de Freund.

- Antigènes membranaires (S2)

Par injection : 60 $\mu$g d'antigènes membranaires (obtenus à l'Exemple 2) dans 75 $\mu$l de PBS 10 mM pH 7,2 + 75 $\mu$l d'adjuvant incomplet de Freund.

c) Immunisation par les antigènes de cerveau de souris Swiss (productrices des kystes) :

Un cerveau de souris Swiss a été broyé, puis soumis de la même façon que les bradyzoïtes à 17 passages à la X-Press. L'immunisation de rats Fisher et de souris Balb/c par l'extrait soluble a été réalisée de la même façon qu'avec les antigènes S1 des bradyzoïtes.

d) Protocole d'infection de rats et de souris :

Infection de rats Fischer

Infection par voie intrapéritonéale par $10^5$ ou $10^7$ tachyzoïtes de la souche RH dans 500 $\mu$l de PBS 10 mM pH 7,2. Infection de rats Nu/Nu [cf SANTORO et al., (C.R. ACAD. SCI. (1987), 304, 297-300)]

Infection de souris Balb/C

Infection par voie intrapéritonéale à raison de 5 à 30 kystes de la souche "chronique" 76 K dans 250 $\mu$l de PBS 10 mM, pH 7,2.

e) Sérums humains

741 sérums, recueillis au Laboratoire Saint-Camille à Lille, aux fins de diagnostic, ont été caractérisés par les tests sérologiques classiques : agglutination directe avant et après traitement par le mercapto-2-éthanol (technique de COUZINEAU) et immunofluorescence indirecte à l'aide d'un sérum de chèvre anti-immunoglobulines humaines G-A-M (IPP, technique de WELLER et COONS) et d'un sérum de chèvre anti IgM humaines (IPP, technique de REMINGTON).

Les Inventeurs ont ainsi répertorié 139 sérums "aigus", caractérisés par la présence d'IgM spécifiques reconnues par au moins deux méthodes sur les trois utilisées, 462 sérums "chroniques", dépourvus d'IgM spécifiques, et 140 sérums négatifs par toutes les techniques.

f) Immunoprécipitation des antigènes

f).1 - Les échantillons sériques, préalablement stérilisés par filtration sur membrane Millipore® (0,22 micromètre), sont aliquotés par 10 microlitres en tubes siliconés (Eppendorf). On ajoute dans ces tubes 500 microlitres de tampon d'adsorption (Tris 10 mM, EDTA 2 mM, NaCl 0,15 M, Nonidet P40 0,5 %, contenant 100 unités Kallikréine d'aprotinine par millilitre (équivalent à 0,11 unité TIU) et ajusté à pH 7,4), puis la dose adéquate d'antigène marqué calculée en fonction du nombre de cpm dans la préparation initiale (40 000 cpm pour les ES marqués à la méthionine $^{35}$S, 250 000 cpm pour les antigènes cytoplasmiques ou membranaires marqués à la méthionine $^{35}$S, 400 000 cpm pour les antigènes des tachyzoïtes et des bradyzoïtes marqués à $^{125}$I et 60 000 cpm pour les produits de traduction marqués à la méthionine $^{35}$S) On laisse ensuite en agitation à température ambiante pendant trois heures.

f).2 - On prépare d'autre part une suspension de protéine A-Sépharose® (Pharmacia) : on laisse gonfler une heure la protéine A-Sépharose® dans le tampon d'adsorption, à raison de 10 milligrammes de produit par échantillon à analyser. On répartit la suspension dans une nouvelle série de tubes siliconés qui sont séparés en deux groupes : le premier ne subit pas de manipulation dans l'immédiat et correspond aux échantillons pour lesquels la protéine A ne doit pas être prétraitée, tandis que le second correspond aux échantillons dans lesquels on veut capter, outre les anticorps de classe IgG qui se fixent d'eux-mêmes sur la protéine A, les anticorps de classe IgM dont la capture nécessite un artifice. Les tubes de ce second groupe sont donc centrifugés (1000 tours pendant 1 minute) et le surnageant éliminé. On Ajoute au culot 200 microlitres de tampon d'adsorption et 50 microgrammes d'un anticorps de classe IgG et de spécificité anti-IgM. La fixation de cet anticorps est assurée par un temps de contact d'une heure sous agitation à température ambiante. Les billes de protéine A-Sépharose® sont ensuite lavées deux fois dans le tampon d'adsorption.

Juste avant utilisation, les tubes des deux groupes sont centrifugés et le surnageant éliminé. On transfère dans ces tubes les immunoprécipités et on laisse en contact pendant une nuit à 4°C sous agitation douce.

Un cycle de 8 lavages par 1 millilitre de tampon d'adsorption est alors effectué. On vérifie la qualité des lavages en mesurant la radioactivité du 8ème surnageant ; de nouveaux lavages sont effectués si celle-ci est trop élevée. On ajoute alors à tous les culots 60 microlitres d'un tampon TRIS 125 mM, SDS 3 %, saccharose 20 %, bleu de bromophénol 0,1 % et $\beta$-mercaptoéthanol 20 %.

Après agitation, les tubes sont soumis à deux ébullitions successives de 1 min. 30, séparées par un nouveau temps d'agitation énergique (Vortex)®.

On centrifuge ensuite tous les tubes à 3000 tours par minute pendant 15 minutes et on transfère les surnageants contenant les immunoprécipités dissociés, dans une nouvelle série de tubes. On mesure sur 5 microlitres la radioactivité de chacun des surnageants, ce qui donne une idée approximative de la quantité piégée par les anticorps sériques. L'ensemble des échantillons est alors soumis à une électrophorèse en gel de polyacrylamide.

g) Electrophorèse (SDS-PAGE) des immunoprécipités et autoradiographie.

Les électrophorèses en gels de polyacrylamide (SDS-PAGE) ont été réalisés selon le protocole décrit par LAEMMLI [(1970) Nature (London) 256, 495-497] dans un appareil à électrophorèse vertical BioRad®.

Les échantillons traités sont séparés dans un gel homogène de polyacrylamide 10 % (dimensions : 14 x 16 cm). L'électrophorèse s'effectue sous un courant de 7mA durant 16 à 18 heures dans un tampon TRIS-glycine pH 8,3 contenant 1 % de SDS.

Après fixation des protéines par un mélange de méthanol et d'acide acétique, le gel est déshydraté sur papier Whatman® (sécheur sous vide, BioRad®). Si les antigènes recherchés sont marqués à la méthionine $^{35}$S, le gel est imprégné pendant 20 minutes d'une émulsion photographique (Amplify® AMERSHAM).

L'autoradiographie des gels se fait contre un film Kodak® X-Omat RP et les films sont révélés après une exposition de 3 (antigènes marqués à $^{125}$I) à 6 (antigènes marqués à la méthionine $^{35}$S) jours à -70°C.

h) Calcul des masses moléculaires des antigènes.

Les gels sont étalonnés grâce à un mélange de protéines précolorées de masse moléculaire connue (commercialisé par BRL, USA). on mesure la distance parcourue dans le gel par ces protéines au cours de l'électrophorèse. Les valeurs obtenues permettent d'établir la droite d'étalonnage d = f(log MM) et donc de déterminer la masse moléculaire des protéines présentes dans les échantillons analysés.

Les masses moléculaires des antigènes mentionnés dans le tableau qui va suivre représentent une moyenne établie à partir d'une trentaine de gels.

TABLEAU I

**ANTIGENES ES RECONNUS AU COURS DE LA TOXOPLASMOSE HUMAINE**

Masse moléculaire                    Spécificité

$\left.\begin{array}{l}185\ 000 \\ 170\ 000 \\ \underline{155\ 000}\end{array}\right\}$ Reconnus au cours de la phase chronique

105 000 ⟶ marqueur de la phase chronique    "doublet"caracté-

98 000 ⟶ marqueur de la phase AIGUE*        risant la phase
                                            chronique

84 000 ⟶ marqueur transitoire de la phase chronique

                        marqueur de la phase aiguë,

$\underline{68\ 000}$ ⟶        de spécificité moindre que la 95 kDa

                        car faiblement reconnue par les sérums négatifs

$\left.\begin{array}{l}\underline{57\ 000} \\ 53\ 000 \\ \underline{45\ 000} \\ 43\ 000 \\ \underline{39\ 000} \\ \underline{35\ 000} \\ 34\ 000 \\ 31\ 000 \\ 30\ 000 \\ \underline{26\ 000} \\ \underline{25\ 000}\end{array}\right\}$ Reconnus au cours de la phase chronique

                        marqueur tardif de la phase chronique ;

$\underline{24\ 000}$ ⟶        marqueur d'immunité protectrice

20 100

\* La 98 kDa, présente tout au long de l'évolution de la maladie, est le premier antigène étroitement spécifique à être reconnu.

**EXEMPLE 4 - Mise en évidence de la reconnaissance des Antigènes ES par des sérums d'animaux.**

Des antigènes ES marqués à la $^{35}$S-méthionine (comme décrit à l'Exemple 1.b) immunoprécipités par différents sérums d'animaux sont reconnus par ceux-ci comme indiqué dans le Tableau II ci-après.

Dans ce Tableau, les chiffres soulignés correspondent à des bandes majeures.

Les chiffres qui figurent dans ledit Tableau se rapportent aux masses moléculaires, en daltons, des antigènes ES reconnus.

## Tableau II

### BILAN DES ESA ($S^{35}$Met) RECONNUS PAR DES SERUMS

| HUMAIN Chronique | SOURIS Chronique | SOURIS anti-ES | RAT INFECTE | RAT Anti-ES | RAT NUDE INFECTE |
|---|---|---|---|---|---|
| 185 000 | | | 185 000 | | 185 000 |
| 170 000 | 170 000 | | 170 000 | 170 000 | |
| 155 000 | 155 000 | 155 000 | | | |
| 98-105000 | 98-105000 | / | 98-105000 | 98-105000 | 98-105000 |
| (doublet) | | | | | |
| 84 000 | 84 000 | 84 000 | 84 000 | 84 000 | |
| 68 000 | 68 000 | 68 000 | 68 000 | 68 000 | 68 000 |
| | | | | (faible) | |
| | | 65 000 | | | |
| | 62 000 | 62 000 | | | |
| 57 000 | 57 000 | | 56 000 | 55 000 | 55 000 |
| 53 000 | | 53 000 | | | |
| 45 000 | | | 45 000 | 45 000 | |
| 43 000 | | 43 000 | 42 000 | 42 000 | 42 000 |
| 39 000 | | | | 39 000 | 39 000 |
| 35 000 | 35 000 | 35 000 | 35 000 | 35 000 | |
| 34 000 | 34 000 | | 34 000 | 34 000 | 34 000 |
| 31 000 | | 31 000 | 31 000 | 31 000 | 31 000 |
| | | | | | (faible) |
| 30 000 | | | | | |
| 26 000 | | | | | |
| 25 000 | 25 000 | 25 000 | 25 000 | 25 000 | 25 000 |
| | | | | | (faible) |
| 24 000 | | | 24 000 | 24 000 | 24 000 |
| 20 100 | | | | | |

Antigènes membranaires majeurs des tachyzoïtes ($^{125}$I)reconnus par des sérums de rats et de souris immunisés par les antigènes ES
$$\left\{ \begin{array}{l} 43\ 000 \\ 30\ 000 \end{array} \right.$$

**EXEMPLE 5 - Induction d'anticorps de classe IgE et leur rôle dans la toxoplasmose expérimentale du rat.**

Chez le rat Fischer normal, comme chez la plupart des hôtes mammifères non-immunodéficients, l'infection par Toxoplasma gondii induit une immunité permanente.

Les mécanismes de la mémoire immunitaire sont connus pour être T-dépendants et nécessitent pour être entretenus la persistance de formes enkystées (bradyzoïtes) cérébrales.

Cependant, l'ensemble des mécanismes responsables de l'induction de cette immunité permanente sont mal connus.

Chez le rat Fischer Nu/Nu, qui représente un modèle de terrain immunocompromis, $10^5$ tachyzoïtes de la souche RH de Toxoplasma gondii injectés I.P. sont suffisants pour tuer 100 % des animaux en 12 à 15 jours.

Des rats Fischer de 10-12 semaines ont été infectés par $10^7$ tachyzoïtes de la souche RH de Toxoplasma gondii. De façon à mieux cerner le rôle des antigènes excrétés/sécrétés (ES), les animaux ont été inoculés soit par des tachyzoïtes vivants, soit par des tachyzoïtes irradiés (10 000 rads délivrés en 10 mn), soit par des antigènes ES préparés comme décrit précédemment.

L'augmentation du taux d'IgE spécifiques dans le sérum (mesuré par radioallergosorbent test ou RAST) et à la surface de certaines cellules sanguines (analysé par cytométrie de flux) a été suivie à intervalles réguliers.

De plus, le pouvoir protecteur de ces IgE spécifiques a été étudié par transfert chez les rats immunodéprimés Nu/Nu avant infection par $10^5$ Toxoplasma gondii.

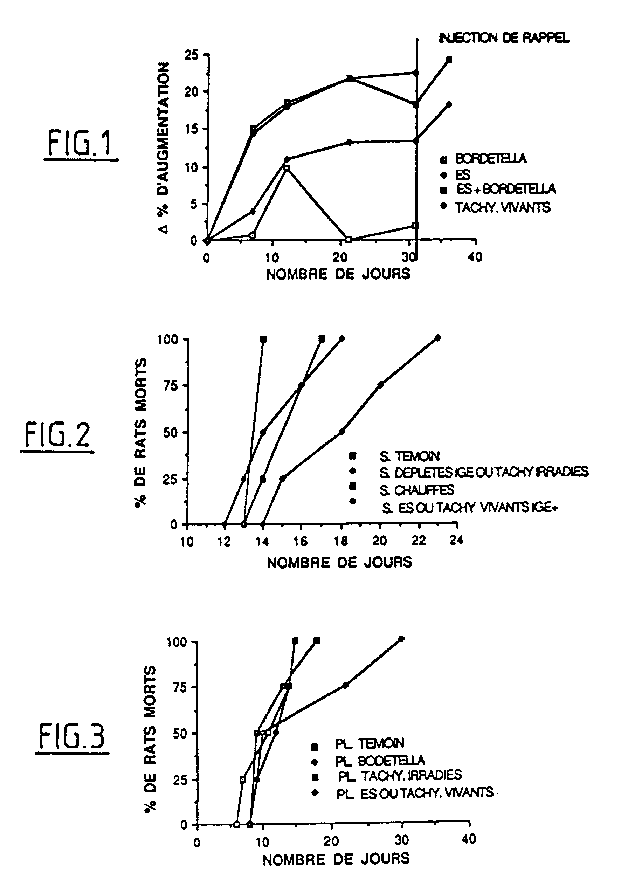

La figure 1 annexée montre la cinétique d'apparition des IgE sériques chez les rats Fischer normaux après différents traitements.

Les tachyzoïtes vivants induisent une réponse IgE spécifique. Cette réponse est extrêmement précoce et peut être détectée dès le 7ème pour après l'infection. La réponse maximum est observée au 21ème jour. L'injection d'antigènes ES à des rats normaux entraîne également une réponse IgE significative.

On peut noter que les antigènes ES induisent le même type de réponse mais moins intense. Néanmoins, après injection de rappel un mois après la première injection, l'augmentation des IgE spécifiques est beaucoup plus importante. On peut noter que l'utilisation d'adjuvants de la réponse IgE (Bordetella pertussis) avec les ES induit une augmentation significative des IgE au cours de la réponse primaire alors que l'injection de l'adjuvant seul n'entraîne pas de réponse spécifique.

La figure 2 annexée montre le résultat des transferts des différents types de sérums chez les rats Fischer Nu/Nu. Tous les sérums utilisés ont été prélevés pendant la réponse primaire au 2e jour.

Lorsque les animaux recoivent 2 ml de sérums riches en IgE spécifiques provenant de rats normaux infectés par des tachyzoïtes vivants ou immunisés par des antigènes ES, on peut noter que l'augmentation du délai de survie est significative par rapport aux animaux qui ont reçu des sérums provenant de ra s immunisés par des tachyzoïtes irradiés ou ayant reçu du sérum physiologique.

Cette augmentation de survie est effectivement due aux IgE car leur dépletion par immunoadsorption ramène la durée de survie à des valeurs proches de celles des contrôles.

La figure 3 annexée montre que des plaquettes provenant d'animaux infectés par des tachyzoïtes vivants ou immunisés par les ES confèrent aux rats Nu/Nu le même type de protection que des sérums des animaux correspondants. On peut également noter que les plaquettes provenant d'animaux immunisés par des tachyzoïtes irradiés ne protègent pas significativement les rats Nu/Nu.

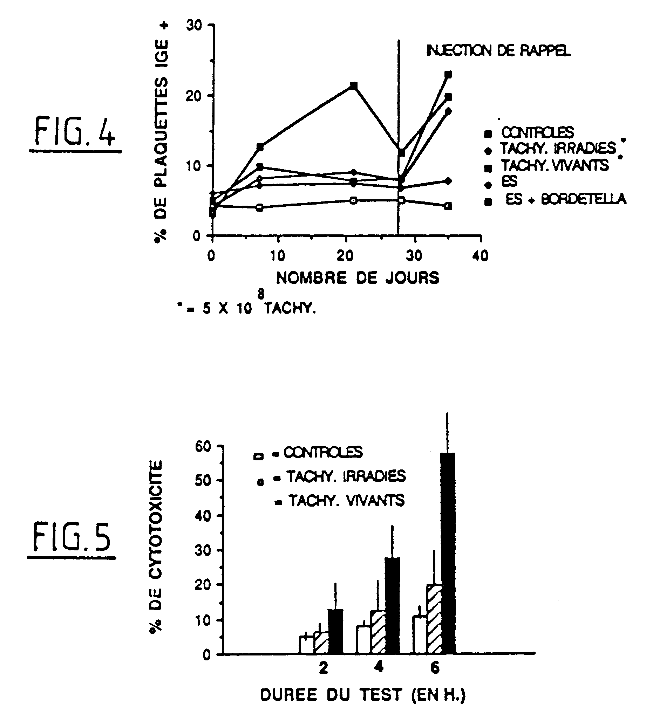

La figure 4 annexée montre que la cinétique d'apparition des IgE à la surface des plaquettes est comparable à celle des IgE sériques. Les taux d'IgE à la surface des plaquettes varient suivant le type d'antigène utilisé de la même façon que les IgE sériques. Le délai de survie est dose-dépendant et une dose de 5 x $10^7$ cellules est suffisante pour entraîner une protection significative.

La protection conférée par les plaquettes apparaît être liée à des phénomènes de type antibody-dependent cell cytotoxicity (ADCC). En effet (cf. figure 5 annexée), on peut observer que des plaquettes provenant d'animaux normaux infectés par des tachyzoïtes vivants ou immunisés par des ES, mais non par des tachyzoïtes tués, sont capables de détruire des tachyzoïtes in vitro. Les mécanismes de cette destruction ne sont pas encore élucidés.

**EXEMPLE 6 - Réponse humorale contre les antigènes ES au cours des phases aiguë, subaiguë et chronique de la toxoplasmose humaine.**

Technique utilisée : radioimmunoprécipitation et analyse des immunoprécipités en SDS-PAGE (gel de polyacrylamide 10 %).

Antigènes : antigènes ES, cytoplasmiques et membranaires des tachyzoïtes marqués par la méthionine $^{35}$S.

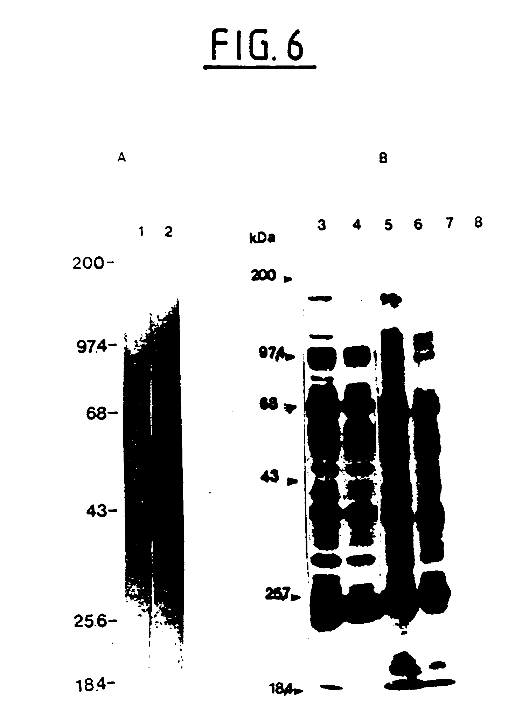

Sérums utilisés : sérums humains prélevés au cours des différentes périodes de la maladie : aiguë, subaiguë, chronique. Les résultats obtenus sont représentés à la figure 6 annexée.

- Les antigènes ES marqués ont été immunoprécipités par des sérums humains prélevés :
  - en phase aiguë (piste 1)
  - en phase subaiguë (piste 2)

- en phase chronique (pistes 3 et 4)

  et par des sérums négatifs (pistes 7 et 8).
- Des antigènes cytoplasmiques (extrait soluble de tachyzoïtes lysés par l'eau) et membranaires (extraits par le NP40) ont à titre comparatif été immunoprécipités par des sérums chroniques :
  - piste 5 : antigènes cytoplasmiques
  - piste 6 : antigènes membranaires.
- Les premiers antigènes qui sont reconnus sont les antigènes 68 et 98 KDa, présents en phases aiguë et subaiguë (partie A)
- En période chronique (partie B), les sérums de patients reconnaissent des antigènes de masse approximative 105, 84, 57, 45, 39, 35, 25 et 24 kDa.

  La bande 105 kDa forme avec la bande 98 kDa un "doublet" spécifique de la phase chronique.

**EXEMPLE 7 - Mise en évidence (après marquage par $^{125}$I) d'un antigène 43 kDa commun aux tachyzoïtes et aux bradyzoïtes,excrété-sécrété par les tachyzoïtes.**

Antigènes immunoprécipités :
- extrait membranaire de tachyzoïtes marqué par $^{125}$I (Iodo-Gen)
- antigènes solubles de bradyzoïtes (S1) marqués par $^{125}$I (chloramine T)
- antigènes membranaires de bradyzoïtes (S2) extraits par le CHAPS, puis marqués par $^{125}$I (chloramine T).

Sérums utilisés :
- sérum de lapin immunisé par les antigènes ES
- sérum de souris Balb/C immunisée par les antigènes membranaires (S$_2$) des bradyzoïtes
- sérum de souris Balb/C immunisée par des antigènes de cellules de cerveau de souris Swiss (les bradyzoïtes, provenant de souris Swiss, sont contaminés par des cellules de cerveau).

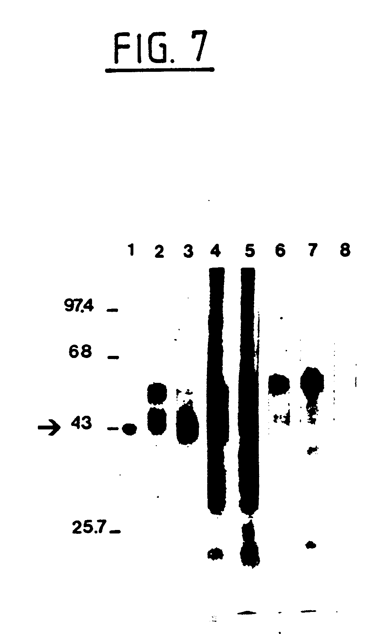

Les résultats obtenus sont représentés à la figure 7 annexée :

Pistes 1 et 2 : Immunoprécipitation par un sérum de lapin anti-ES :
- d'antigènes membranaires de tachyzoïtes (piste 1)
- d'antigènes membranaires (S2) de bradyzoïtes (piste 2) Pistes 3 à 5 : Immunoprécipitation par un sérum de souris Balb/C anti⁻bradyzoïtes (fraction S2)
- d'un extrait membranaire de tachyzoïtes (piste 3)
- d'un extrait soluble (fraction S1) de bradyzoïtes (piste 4)
- d'un extrait membranaire (fraction S2) de bradyzoïtes (piste 5)

Pistes 6 à 8 : Immunoprécipitation par un sérum de souris Balb/C anti-cerveau
- d'un extrait soluble (S1) de bradyzoïtes (piste 6)
- d'un extrait membranaire (S2) de bradyzoïtes (piste 7)
- d'un extrait membranaire de tachyzoïtes (piste 8). L'antigène 43 kDa des tachyzoïtes et des bradyzoïtes reconnu par les sérums anti-ES et anti-bradyzoïtes l'est également par les sérums humains.

L'antigène d'environ 55kDa, reconnu par les sérums de souris anti-cerveau ne semble pas spécifique des bradyzoïtes.

**EXEMPLE 8 - Mise en évidence (après marquage par la Met $^{35}$S) d'un antigène d'environ 25 kDa commun aux tachyzoïtes et aux bradyzoïtes, excrété-sécrété par les tachyzoïtes.**

- Antigènes immunoprécipités (marqués par la méthionine $^{35}$S)
  - Antigènes ES
  - Antigènes cytoplasmiques des tachyzoïtes (fraction $H_2O$)
  - Antigènes totaux des tachyzoïtes (traitement par le CHAPS des parasites entiers).
- Sérums utilisés
  - sérums de souris Balb/C immunisées par les antigènes solubles (S1) ou membranaires (S2) des bradyzoïtes
  - sérum de souris Balb/C immunisées par un extrait de cellules de cerveau de souris Swiss
  - sérum de rats Fischer immunisés par les antigènes solubles (S1) ou membranaires (S2) des bradyzoïtes.

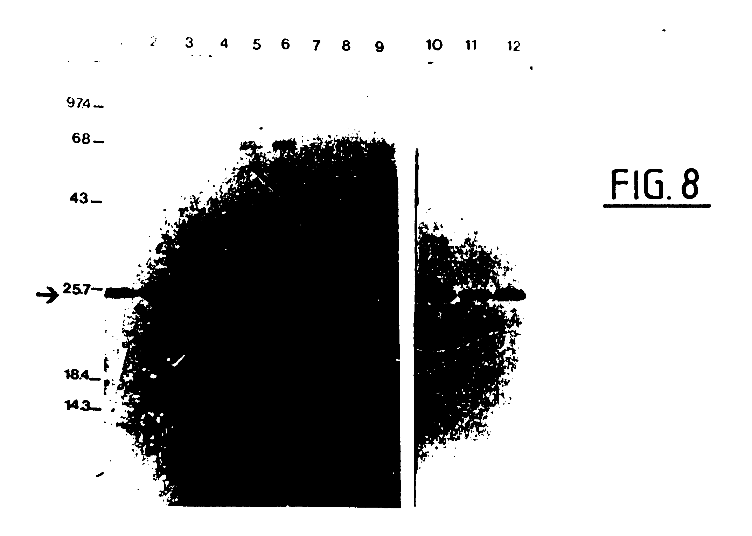

Les résultats obtenus sont représentés à la figure 8 annexée.
- Pistes 1 à 3 : Immunoprécipitation par un sérum de souris Balb/C anti-bradyzoïtes (fraction S2)
  - d'antigènes ES (piste 1)

- d'antigènes cytoplasmiques (fraction $H_2O$) des tachyzoïtes (piste 2)
- d'antigènes totaux (fraction CHAPS) des tachyzoïtes piste 3).
- Pistes 4 à 6 : Immunoprécipitation par un sérum de souris Balb/C anti-bradyzoïtes (fraction S1)
  - d'antigènes ES (piste 4)
  - d'antigènes cytoplasmiques des tachyzoïtes (piste 5)
  - d'antigènes totaux des tachyzoïtes (piste 6).
- Pistes 7 à 9 : Immunoprécipitation par un sérum de souris Balb/c anti-cellules de cerveau de souris Swiss
  - d'antigènes ES (piste 7)
  - d'antigènes cytoplasmiques des tachyzoïtes (piste 8)
  - d'antigènes totaux des tachyzoïtes (piste 9).
- Pistes 10 à 12 : Immunoprécipitation par un sérum de rat Fischer anti-bradyzoïtes (fraction S2)
  - d'antigènes totaux de tachyzoïtes (piste 10)
  - d'antigènes cytoplasmiques de tachyzoïtes (piste 11)
  - d'antigènes ES (piste 12).

**EXEMPLE 9 - Mise en évidence de la nature glycoprotéique de l'antigène 43** kDa.

Les antigènes de surface des tachyzoïtes marqués par $^{125}I$ (Iodo-Gen) ont été absorbés sur ConA-Sepharose® ou sur WGA-Ultrogel® en procédant comme suit :

La fixation sur lectines a été réalisée suivant le protocole décrit par COLMAN et Al. [(1981) EUR. J. BIOCHEM., 113, 339-348]

Tampon de couplage

150 mM NaCl
0,7 mM $MgCl_2$
1 mM dithiothreitol
0,7 mM $MnCl_2$
0,7 mM $CaCl_2$
0,05 % sodium dodécyl sulfate
20 mM Tris/HCl, pH 7,5
Lectines : Concanavaline A : ConA-Sepharose® (Pharmacia)
Wheat germ agglutinine = WGA-Ultrogel® (IBF)
Antigènes :
  1) extrait membranaire (NP40) de tachyzoïtes marqués par $^{125}I$ (Iodogen)
  2) extrait $H_2O$ de tachyzoïtes marqués par la méthionine $^{35}S$
  3) extrait membranaire (CHAPS) de tachyzoïtes marqués par la méthionine $^{35}S$

Technique utilisée

Les réactions sont effectuées dans des tubes Eppendorf.
- Addition à 150 $\mu l$ d'extrait 1) ou à 200 $\mu l$ des extraits 2) et 3) de : 1 ml de tampon de couplage
- 250 $\mu l$ de gel (ConA-Sepharose® ou WGA-Ultrogel®) pré équilibré dans le tampon de fixation.

Après 15 mn de contact à température ambiante avec des agitations fréquentes, les tubes sont centrifugés. Le surnageant contient les antigènes non retenus. Le culot est ensuite lavé 3 fois dans le tampon de couplage, puis extrait par 3 x 200 $\mu l$ de tampon de couplage contenant soit 0,4 M de O-Méthyl-$\alpha$-D-mannopyranoside (IBF), dans le cas de la fixation sur Concanavaline A, soit 0,2 M de N-acétyl-glucosamine (Sigma)® dans le cas de la WGA. Ces trois extractions successives constituent les éluats 1, 2 et 3.

La radioactivité est comptée dans les divers échantillons, puis les antigènes non traités, les fractions non retenues et les éluats (1 + 2) sont immunoprécipités par différents sérums.

Les antigènes de départ ainsi que les fractions retenues sur les deux lectines (éluats) ont été immunoprécipités par un sérum de lapin sain et un sérum de lapin anti-ES.

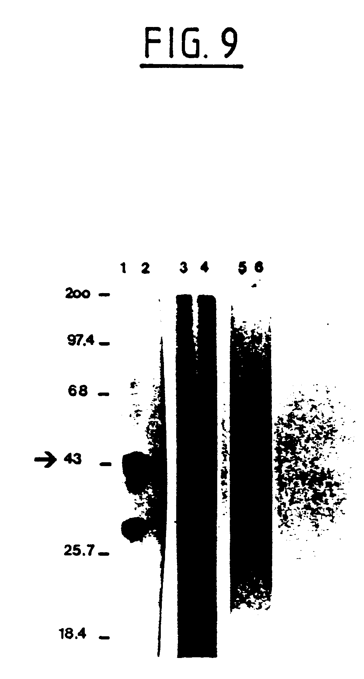

La figure 9 montre :
- pistes 1, 3 et 5 : Immunoprécipitation par un sérum de lapin anti-ES.
- pistes 2, 4 et 6 : Immunoprécipitation par un sérum de lapin sain.

EP 0 301 961 B1

- pistes 1 et 2 : antigènes membranaires de tachyzoïtes, avant traitement.
- pistes 3 et 4 : éluat ConA-Sépharose® (antigènes fixés).
- piste 5 et 6 : éluat WGA-Ultrogel®.

Les pistes 3 et 5 montrent que l'antigène 43 kD se fixe sur la concavaline A et (faiblement) sur la WGA (agglutinine de germe de blé), démontrant ainsi sa nature glycoprotéique.

**EXEMPLE 10 - Préparation des produits de traduction des ARNs messagers de tachyzoïtes.**

a) Extraction de l'ARN total :

On utilise la méthode de AUFFRAY et ROUGEON [(1980) Eur. J. Biochem, 107, 303]. Les tachyzoïtes sont lysés par l'addition (10 fois le volume du culot parasitaire) d'une solution de LiCl 3M, Héparine 200 $\mu$g/ml ; acétate de Na 10 mM, pH 5 ; SDS 0,1 %.

Après homogénéisation au Potter et conservation à + 4°C toute la nuit pour précipiter les ARNs, le lysat est centrifugé à 16 300 g pendant 40 minutes à + 4°C. Le culot est lavé deux fois par resuspension dans une solution de LiCl 4M, Urée 8M, puis de nouveau centrifugé à 16 300 g pendant 40 minutes à + 4°C. Le culot est ensuite dissous dans de l'$H_2O$ RNAse-free. Cette solution est amenée à 0,1 M en acétate de sodium pH 5, SDS 0,1 %, pour être extraite successivement par un volume de phénol saturé en $H_2O$, par un volume de phénol-chloroforme. La phase aqueuse est finalement amenée à 0,2 M en acétate de sodium pH 5 pour précipiter l'ARN par 2,5 volumes d'éthanol absolu à - 20°C pendant une nuit.

b) Purification des ARNs messagers :

On utilise la méthode de ARNEMANN et Al. [(1977), Nucl. Acids Res. 4, 4023-4026].

Cette méthode consiste à séparer les ARNs poly A+ de l'ARN total par 2 chromatographies successives sur oligo (dT) cellulose.

c) Traduction in vitro des ARNs m dans le lysat de réticulocytes de lapin /PELHAM et JACKSON, (1986), Eur. J.

Biochem, 67, 247/. Les ARNs m (0,5 $\mu$g) sont traduits dans 30 $\mu$l de lysat de réticulocytes (N-150 AMERSHAM), en présence d'acétate de potassium (110 mM), d'acétate de magnésium (1mM), d'un cocktail d'aminoacides sans L-méthionine (50 mM) et de 50 $\mu$Ci de $^{35}$S méthionine (AMERSHAM).

d) Immunoprécipitation des produits de traduction

L'immunoprécipitation est réalisée comme décrit à l'Ex. 3-d) ci-dessus ; brièvement, après incubation de produits de traduction (60 000cpm) avec les sérums (10$\mu$l), les immuncomplexes sont adsorbés sur 10 mg de protéine A-Sépharose® (Pharmacia), puis élués pour être analysés en SDS-PAGE (LAEMMLI, 1970, NATURE, 227, 680).

**EXEMPLE 11 - Immunoprécipitation des produits de traduction d'ARNm de tachyzoïtes.**

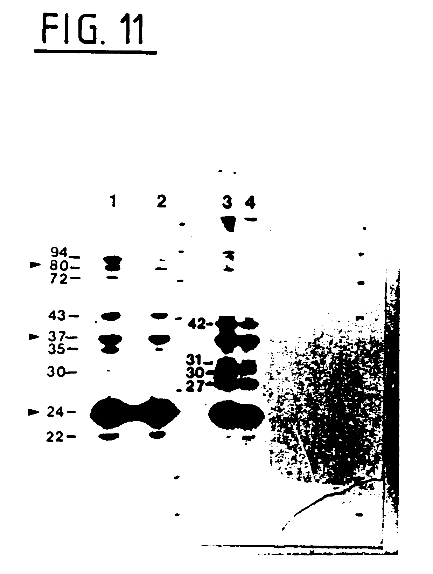

Les ARNm sont' préparés par la méthode décrite à l'Exemple 10 ci-dessus (pistes 1 et 3 de la figure 11 annexée) ou par la méthode de CHIRGWIN et Al. [(1978) Biochemistry, 18, 5294-5299] (pistes 2 et 3 de la figure 11). Cette figure montre également les profils des produits de traduction immunoprécipités par le sérum humain de la phase chronique de l'infection (pistes 3 et 4).

Les produits de traduction 24 kDa,37 kDa et 80 kDa reconnus par les deux sérums sont indiqués par une flèche.

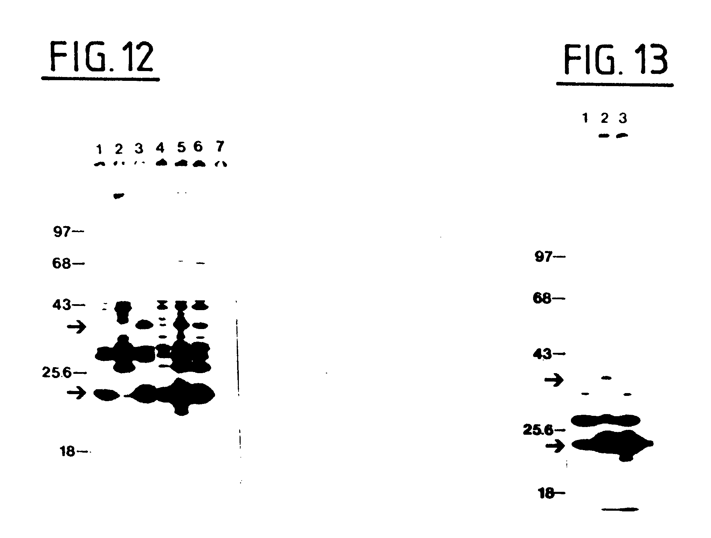

L'immunoprécipitation des produits de traduction par une série de sérums humains des phases chronique et aiguë est en outre représentée comparativement à la figure 12 annexée ; en particulier leur immunoprécipitation :

- par une série de sérums humains de la phase chronique (IgG) (pistes 1, 3, 4, 5, 6),
- par un sérum humain en fin de phase aiguë (IgG + IgM) (piste 2),
- par un sérum humain négatif (piste 7).

La reconnaissance des produits de traduction 24 kDa et 37 kDa par tous les sérums chroniques est symbolisée par une flèche. Ces deux antigènes ne sont pas reconnus par le sérum de la phase aiguë.

**EXEMPLE 12 - Caractérisation de l'antigène 24 kDa.**

Mise en évidence de la nature protéique de l'antigène 24 kDa.

Un extrait total de tachyzoïtes (extraction CHAPS) marqués par la méthionine $^{35}$S a été adsorbé sur ConA-Sépharose® ou sur WGA-Ultrogel®. L'extrait de départ ainsi que les éluats des deux lectines ont été immunoprécipités par un sérum de lapin anti-ES.

17

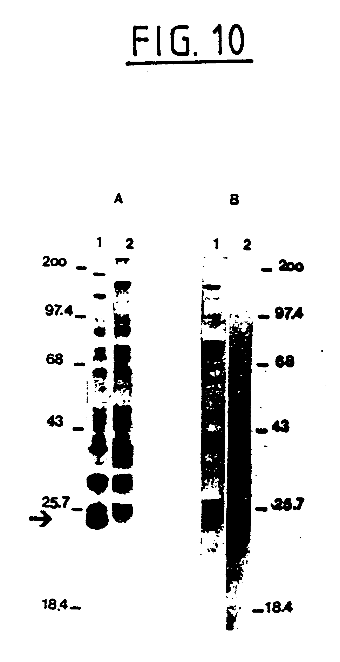

Les résultats obtenus sont représentés à la figure 10A annexée.

Aucun antigène marqué par la méthonine $^{35}$S n'a été retenu sur WGA.

Piste 1 : Immunoprécipité de l'extrait total de tachyzoïtes,

Piste 2 : Immunoprécipité de l'éluat ConA-Sépharose®.

Certains antigènes (notamment ceux de masse environ 170 kDa, 39 kDa et 25 kDa) ont des groupements glycosylés qui se fixent à la Concanavaline A. L'antigène 24 kDan'est par contre pas retenu.

Figure 10B : cette figure met en évidence l'identité de masse moléculaire entre l'antigène ES de 24 kDa et le produit de traduction majeur des ARNm de tachyzoïtes.

Piste 1 : Immunoprécipitation d'antigènes ES marqués par la méthionine $^{35}$S, par un sérum de souris anti-ES.

Piste 2 : Immunoprécipitation des produits de traduction marqués par la méthionine $^{35}$S par un sérum de lapin anti-ES.

Etant donné d'une part la nature protéique de l'antigène 24 kDa(Fig. 10A) et, d'autre part, sa migration voi sine de celle du produit de traduction, l'identité des deux molécules est fortement présumée.

## EXEMPLE 13 - Immunocompétition entre les produits de traduction du tachyzoïte et l'antigène bradyzoïte extrait au SDS 2 % vis-à-vis d'un sérum de lapin anti-ES.

Cette immunocompétition est représentée à la figure 13 annexée dans laquelle :

- la piste 1 : Immunoprécipitation des produits de traduction en présence de 50 $\mu$g d'extrait de bradyzoïtes.
- la piste 2 : Immunoprécipitation des produits de traduction en présence de 50 $\mu$g d'extrait de S. mansoni.
- la piste 3 : Immunoprécipitation des produits de traduction seuls.

L'antigène bradyzoïte est capable de diminuer la fixation des produits de traduction 24 kDa et 37 kDa par des anticorps anti-ES.

EXEMPLE 13 bis - Identification d'antigènes ES d'intérêt diagnostique par immunoprécipitation par des sérums humains prélevés au cours des différentes phases de la maladie.

D'autre part, une étude à visée diagnostique a été réalisée à l'aide d'une sérothèque de 350 sérums bien caractérisés dont certains ont été prélevés au cours des différentes phases de la toxoplasmose et montrent une séroconversion ; d'autres, plus tardifs, permettent le suivi de la réponse anticorps ; d'autres encore, correspondant à des "couples" maman-bébé, ont été prélevés pendant les périodes pré-, péri- et post-natales.

L'immunoprécipitation par ces sérums des antigènes ES (marqués par la méthionine $^{35}$S) a permis d'établir la cinétique d'apparition des anticorps et de mettre notamment en évidence :

- que les sérums de phase aiguë (IgM) reconnaissent les antigènes ES de façon beaucoup plus intense que les antigènes somatiques ou membranaires ;
- que deux antigènes ES, de masse environ 98000 et 68000, reconnus de façon majeure et très précoce, pourraient être utilisés comme marqueurs de séroconversion ;
- que d'autres antigènes, reconnus de façon beaucoup plus tardive, notamment celui de masse 24000, semblent au contraire constituer de réels marqueurs de l'immunité dans la toxoplasmose.

Cet antigène de masse 24000, qui ne semble pas glycosylé (pas de fixation sur la Concanavaline A et la WGA) semble correspondre au produit de traduction d'ARN de tachyzoïtes de masse 24. Ce produit de traduction est en effet également immunoprécipité par les sérums tardifs d'infection humaine et non par les sérums précoces (IgM + IgG).

## EXEMPLE 14 - Clonage des produits de traduction.

### a) Préparation de la banque d'ADNc à partie de l'ARNm de tachyzoïtes

La synthèse de l'ADNc est réalisée selon la méthode de GUBLER et HOFFMAN [(1983) GENE, 25, 263]. Le premier brin d'ADN complémentaire est synthétisé par l'action de la transcriptase reverse de AMV. L'ADN, rendu double brin par l'action conjointe de la RNAse H et de la polymérase I, est ensuite traité à la T$_4$ DNA polymérase pour rendre ses extrétés franches. Après méthylation par l'enzyme EcoRI méthylase, on ajoute des "linkers" EcoRI aux extrémités des ADNc par l'action de la T4 DNA ligase. Les ADNc sont ensuite digérés par l'enzyme EcoRI et sont purifiés par chromatographie sur une colonne ACA34 (0,2 x 0,30 cm) [WATSON, C.J. et JACKSON, J.F. (1985) In Glover DM ed. : "DNA cloning" vol.

EP 0 301 961 B1

I. A practical approach : Oxford, Washington : IRL Press, p. 49]. On insère ensuite les ADNc (500 à 7000 pb) dans le site EcoRI du phage GT11 [HUYNH et Al.; (1985) In Glover D.M., ed. "DNA Cloning" vol. I. A practical approach : Oxford, Washington : IRL Press, p. 49].

Après ligation, les phages recombinants sont empaquetés in vitro. Une banque de $3 \times 10^6$ phages est ainsi constituée.

**b) Criblage de la banque et sélection des candidats "ES"**

Le dérivé du phage λ GT11 permet l'expression de gènes étrangers sous le contrôle du promoteur Lac, après fusion de l'ADNc avec le gène de la β-galactosidase de E. coli (Young and Davis, 1983).

Un échantillon de la banque en λ GT11 est inoculé sur la souche de E. coli Y1090 à une dilution représentant $1.10^4$ phages/boîte de 90 mm de diamètre.

L'induction de l'expression de la protéine sous contrôle du promoteur Lac est déclenchée à 42°C en présence d'isopropyl-β-D-thiogalactopyranoside (IPTG).

Les protéines synthétisées sont absorbées sur filtre de nitrocellulose pour être incubées en présence d'anticorps de lapin anti-ES (obtenus comme décrit à l'Ex. 3.a) ci-dessus).

Les anticorps fixés sont détectés par un second anticorps anti-IgG de lapin marqué à la péroxydase ; ce complexe est ensuite révélé par une coloration avec le 4 chloro-1-naphtol en présence de $H_2O_2$.

Cette détection permet d'identifier les phages recombinants dont l'insertion d' ADNc dirige la synthèse d'une protéine ou fraction de protéine portant les épitopes reconnus par les anticorps anti-ES.

Une première sélection a conduit à l'isolement de 100 clones (ES). Après purification, 20 d'entre eux ont été recriblés différentiellement par deux sérums humains (voir figure 7) : un sérum $IgG_{18}$ reconnaissant les produits de traduction de 24 et 37 kDa; l'autre $IgG_{20}$ ne les reconnaissant pas. 3 clones ES8, ES10 et ES11 sont isolés pour leur reconnaissance par le sérum $IgG_{18}$.

**c) Caractérisation des clones ES8, ES10, ES11 par sélection d'anticorps**

La méthode de sélection d'anticorps utilisée est celle décrite par Olmsted (1981), J. BIOL. CHEM., 226, 11955.)

Un échantillon de chaque clone est inoculé sur la souche Y1090 de E. coli à raison de $10^4$ à $10^5$ phages/boîte de 90 mm de diamètre.

L'induction de l'expression est réalisée à 42°C, en présence d'IPTG et les protéines synthétisées sont absorbées sur filtres de nitrate de cellulose. Les filtres sont incubés avec un sérum de lapin anti-ES (1/125) pendant 3 heures à la température ambiante.

Après 3 lavages, les anticorps fixés spécifiquement aux protéines de fusion sont décrochés en présence de tampon glycocolle-HCl pH 2,8 ; 0,1 M ; NaCl 0,15 M pendant 3 minutes.

La solution est rapidement neutralisée par du Tris 1M.

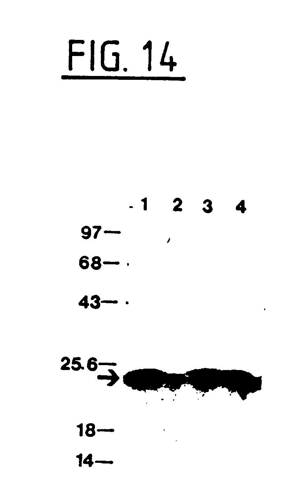

Les anticorps sélectionnés sont concentrés sur 10 mg de protéine A-Sépharose® (Pharmacia) et utilisés pour l'immunoprécipitation des produits de traduction. Les anticorps sélectionnés par les protéines de fusion des 3 clones reconnaissent le produit de traduction 24 kDa, ainsi que cela ressort de la figure 14 annexée qui représente l'immunoprotection des produits de traduction par des anticorps sélectionnés par les clones'ES8, ES10 et ES11.

Piste 1 : Anticorps d'un sérum de lapin anti-ES sélectionnés par le clone ES11.
Piste 2 : Anticorps d'un sérum de lapin hyperimmun sélectionnés par le clone ES11.
Piste 3 : Anticorps d'un sérum de lapin anti-ES sélectionnés par le clone ES10.
Piste 4 : Anticorps d'un sérum de lapin anti-ES sélectionnés par le clone ES-8.

**EXEMPLE 15 - Sous-clonage des "inserts" des clones ES 8, 10 11 dans le vecteur pUC13**

1) Obtention de l'ADN phagique (λGT11)

Les bactériophages correspondant à chacun des clones sont adsorbés sur E. coli Y1090 20 minutes à température ambiante. L'incubat est ensuite étalé sur des boîtes d'agarose en présence d'ampicilline (à raison de 20.000 pfU/boîte).Après une nuit à 37°C, les bactériophages sont élués dans ddu SM (NaCl 100 mM ; $MgSO_4$ $7H_2O$ 8mM ; Tris 50 mM pH 7,5 ; Gélatine 0,01 %).

L'éluat est d'abord traité au chloroforme puis par de la DNAase de la RNAase (1 μg/ml) pour éliminer les constituants bactériens.

Les phages sont ensuite précipités dans du PEG 10 % et NaCl 1,25 M à 4°C.

Une ultracentrifugation à l'équilibre en gradient de CsCl est effectuée pendant 24 heures à 108.000 g (Beckman L8 70 rotor SW 55 Ti).

La bande phagique est récupérée à l'aide d'une aiguille de la solution de phages est dialysée deux fois pendant 1 heure contre du Tampon Tris 10 mM pH 7,4 ; $MgCl_2$ 20 mM NaCl 0,15 M. L'ADN phagique est ensuite obtenu par traitement au Tris HCl 10 mM, EDTA 1 mM et SDS 0,1 % et purifié par une extraction au phénol/chloroforme. Les "inserts" sont ensuite séparés de l'ADN phagique par une digestion EcoRI et migration sur gel d'agarose à bas point de fusion 1,2 %. Après coloration du gel dans une solution de bromure d'éthidium (10 $\mu$g/ml), les fragments de digestion, sont visualisés aux ultraviolets. On peut ainsi déterminer selon la distance de migration la taille des différents "inserts" : l'insert ES8 est de 900 paires de bases, l'insert ES10 est de 1900 paires de bases, l'insert ES11 est de 1300 paires de bases.

Les "inserts" sont ensuite extraits de l'agarose par la technique du "GENECLEAN" (Biol01).

2) Sous-clonage dans pUC13

Le plasmide pUC13 (PHARMACIA) linéarisé par digestion EcoRI possède des extrêmités 5'phosphorylées pour minimiser la recirculation du plasmide. La réaction de ligation entre le pUC13 et l'insert est effectuée en utilisant 1 unité d'ADN ligase du phage T4 (Genofit). Le rapport molaire vecteur/insert doit être de l'ordre de 1. Le mélange de ligation est ensuite utilisé pour la transformation de la souche E. coli JM103, selon la méthode classique de HANAHAN /J. Mol. Biol., (1983), 166, 553/. Les cellules transformées sont étalées sur des boîtes de LB agar en présence d'ampicilline, de XGal et d'IPTG. Les colonies blanches sont celles qui ont intégré les plasmides recombinants.

Pour chacun des clones ES8, ES10 et ES11, nous avons sélectionné une colonie blanche : pUC13, ES8.10, ES10.2,ES 11.7. Carte de restriction des 3 clones pUC13 ES 8.10, ES 10.2, ES 11.7.

Les plasmides recombinants qui dirigent la synthèse de l'antigène 24 KD ont été déposés sous les N° I-675, I-676 et I-677 en date du 16 Juillet 1987 auprès de la CNCM.

1) Préparation de l'ADN plasmidique

Cette étape permet de séparer l'ADN plasmidique de l'ADN bactérien. Elle est réalisée par centrifugation en gradient isopycnique de CsCl selon la méthode de BULHER et TREICH (telle que mise en oeuvre au Service de Biochimie).

2) Digestion par les endonucléases

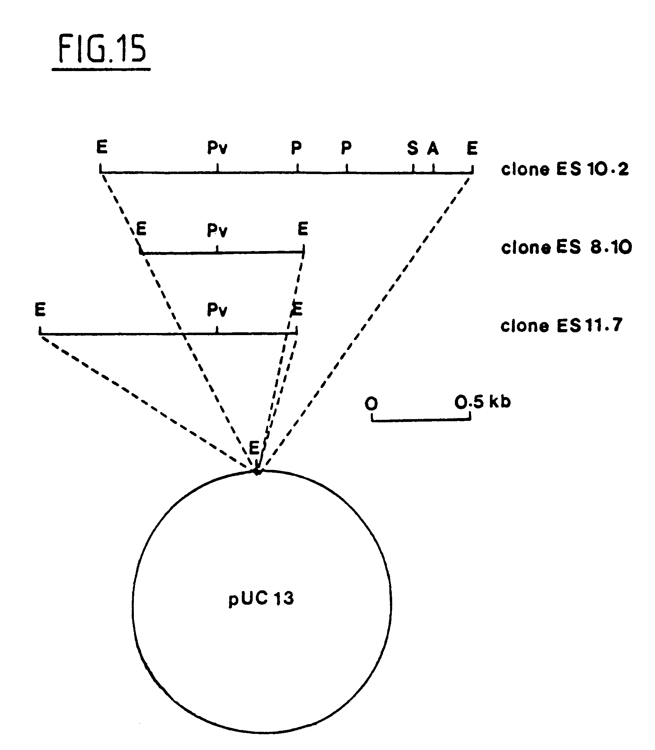

L'ADN plasmidique purifié est ensuite digéré par différentes enzymes de restriction (EcoRI, AccI, PsTI, PvUII et SaCI). Après analyse des fragments de digestion sur gel d'agarose 1,2 %, les cartes de restriction des trois inserts ont été établies et représentées à la figure 15 annexée qui montre le sous-clonage des inserts des 3 clones de TG, ES 8.10 (900 pb), ES 10.2 (1900 pb), ES 11.7 (1300 pb) dans le site EcoRI du vecteur plasmidique pUC13 (2700 pb).

Dans cette figure, A = AccI, E = EcoRI, P = PstI, Pv = PvuII, S = SaCI.

Ainsi que cela ressort de ce qui précède, l'invention ne se limite nullement à ceux de ses modes de mise en oeuvre de réalisation et d'application qui viennent d'être décrits de façon plus explicite ; elle en embrasse un contraire toutes les variantes qui peuvent venir à l'esprit du technicien en la matière, sans s'écarter du cadre, ni de la portée de la présente invention.

**Revendications**

**Revendications pour les Etats contractants suivants : AT, BE, CH, DE, FR, GB, IT, LI, LU, NL, SE**

**1.** Préparation antigénique de *Toxoplasma gondii* (TG), caractérisée :

* en ce qu'elle est susceptible d'être obtenue par :

- incubation d'une suspension de tachyzoïtes de *Toxoplasma gondii* pendant environ 3 heures dans un milieu acellulaire de type RPMI 1640 contenant 10 % de sérum décomplémenté d'origine humaine ou animale ;
- centrifugation de la suspension incubée et récupération des surnageants dépourvus de corps parasitaires et contenant les antigènes d'extraction-sécrétion (antigènes ES) ;
- filtration desdits surnageants, pour l'obtention de ladite préparation antigénique ; et éventuellement
- concentration et congélation à - 70°C de ladite préparation antigénique, et

* en ce qu'elle contient un mélange d'antigènes d'excrétion-sécrétion de tachyzoïtes de TG, capables d'induire une réponse humorale protectrice de type IgE contre la toxoplasmose.

**2.** Préparation antigénique selon la revendication 1, caractérisé en ce qu'elle est constituée par des antigènes d'excrétion-sécrétion de tachyzoïtes de TG, de poids moléculaires respectifs 185 kDa, 170 kDa, 155 kDa, 105 kDa, 98 kDa, 84 kDa, 68 kDa, 57 kDa, 53 kDa, 45 kDa, 43 kDa, 39 kDa, 35 kDa, 34 kDa, 31 kDa, 30 kDa, 26 kDa, 25 kDa, 24 kDa et 20 kDa.

**3.** Antigènes ES de *Toxoplasma gondii,* caractérisés en ce qu'ils sont susceptibles d'être obtenus à partir de la préparation antigénique selon la revendication 1 ou la revendication 2 par :

- immunoprécipitation des différents antigènes ES contenus dans ladite préparation, préalablement radiomarqués, par mise en contact de cette dernière avec un sérum de mammifère, notamment sérum humain ou animal convenable choisi dans le groupe qui comprend les sérums prélevés chez des malades et reconnus comme séropositifs (soit de phase aiguë contenant des IgM spécifiques, soit de phase subaigüe contenant à la fois des IgM et des IgG, soit de phase chronique contenant des IgG) et les sérums obtenus par immunisation par une préparation antigénique selon la revendication 1 ou la revendication 2 et contenant des anticorps anti-ES ;
- piégeage des complexes antigènes-anticorps formés (immunoprécipités) sur des billes de protéine A-Sépharose®, éventuellement traitées de manière à fixer les anticorps de type IgG et les anticorps de type IgM ;
- séparation desdits antigènes ES par centrifugation et récupération des surnageants contenant les immunoprécipités dissociés, et éventuellement
- électrophorèse des immunoprécipités en gels de polyacrylamide.

4. Antigènes ES selon la revendication 3, caractérisés en ce qu'ils présentent un poids moléculaire 185 kDa, 170 kDa, 155 kDa, 105 kDa, 57 kDa, 53 kDa, 45 kDa, 43 kDa, 39 kDa, 35 kDa, 34 kDa, 31 kDa, 30 kDa, 26 kDa, 25 kDa et 24 kDa et sont reconnus au cours de la phase chronique.

5. Antigène ES selon la revendication 3, caractérisé en ce qu'il présente un poids moléculaire de 105 kDa et en ce que qu'il correspond à un marqueur de la phase chronique de la toxoplasmose.

6. Antigène ES selon la revendication 3, caractérisé en ce qu'il présente un poids moléculaire de 84 kDa et en ce qu'il correspond à un marqueur transitoire de la phase chronique de la toxoplasmose.

7. Antigène ES selon la revendication 3, caractérisé en ce qu'il présente un poids moléculaire de 98 kDa et en ce qu'il correspond à un marqueur de la phase aiguë de la toxoplasmose.

8. Antigène ES selon la revendication 3, caractérisé en ce qu'il présente un poids moléculaire de 68 kDa et en ce qu'il correspond à un marqueur de la phase aiguë de la toxoplasmose.

9. Antigènes ES selon la revendication 3, caractérisés en ce qu'ils sont susceptibles d'être obtenus à la fois par immunoprécipitation de la préparation antigénique selon la revendication 1 ou la revendication 2 :
   - avec un sérum convenable choisi dans le groupe qui comprend les sérums prélevés chez des malades et les sérums obtenus par immunisation par une préparation antigénique selon la revendication 1 ou la revendication 2 et contenant des anticorps anti-ES et
   - avec des sérums obtenus par immunisation par des antigènes de bradyzoïtes, et
   en ce qu'ils correspondent à des antigènes ES communs aux tachyzoïtes et aux bradyzoïtes de TG, de poids moléculaire d'environ 25 kDa-24 kDa.

10. Antigènes ES selon la revendication 3, caractérisés en ce qu'ils sont susceptibles d'être obtenus à la fois par immunoprécipitation de la préparation antigénique selon la revendication 1 ou la revendication 2 :
    - avec un sérum convenable choisi dans le groupe qui comprend les sérums prélevés chez des malades et les sérums obtenus par immunisation par une préparation antigénique selon la revendication 1 ou la revendication 2 et contenant des anticorps anti-ES et
    - avec des sérums obtenus par immunisation par des antigènes de bradyzoïtes, et
    en ce qu'ils correspondent à des antigènes ES communs aux tachyzoïtes et aux bradyzoïtes de TG, de poids moléculaire d'environ 43 kDa et de nature glycoprotéique.

11. Produit de traduction d'ARNm de tachyzoïtes, caractérisé en ce qu'il comprend au moins un antigène ES selon l'une quelconque des revendications 3 à 10 et en ce qu'il est reconnu par des sérums humains de phase chronique ou de phase aiguë de l'infection à *Toxoplasma gondii.*

12. Produit de traduction d'ARNm de tachyzoïtes selon la revendication 11, caractérisé en ce qu'il est susceptible d'être obtenu par traduction *in vitro* des ARNm, extraits de lysats de tachyzoïtes, dans du lysat de réticulocytes, en présence d'un mélange d'aminoacides dépourvus de L-méthionine et comprenant la $^{35}$S-méthionine, en milieu tamponné.

**13.** Procédé de clonage des produits de traduction selon la revendication 11 ou la revendication 12, caractérisé en ce qu'il comprend :
- l'extraction d'ARNm à partir de lysats de tachyzoïtes,
- et la synthèse d'ADNc à partir desdits ARNm de tachyzoïtes précédemment obtenus, par action de la transcriptase réverse d'AMV, la banque d'ADNc ainsi formée étant ensuite criblée pour identifier les phages recombinants dont l'insertion d'ADNc dirige la synthèse d'une protéine ou fraction de protéine portant les épitopes reconnus par les anticorps anti-ES et y sélectionner les clones ES reconnus par des sérums humains, les clones retenus étant caractérisés par sélection d'anticorps.

**14.** Sérum riche en anticorps IgE propres à conférer une protection significative contre la toxoplasmose et en particulier contre les tachyzoïtes, caractérisé en ce qu'il est susceptible d'être obtenu à partir d'animaux immunisés par des préparations antigéniques, des antigènes ES ou des produits de traduction d'ARNm de tachyzoïtes selon l'une quelconque des revendications 1 à 12.

**15.** Anticorps monoclonal, caractérisé en ce qu'il est susceptible d'être produit par des hybridomes résultant de la fusion de cellules d'animaux immunisés par les antigènes ES selon l'une quelconque des revendications 3 à 10 avec des cellules tumorales.

**16.** Vaccin propre à procurer une protection significative contre la toxoplasmose, caractérisé en ce qu'il contient, en tant que constituant actif, une préparation antigénique ou un antigène ES selon l'une quelconque des revendications 1 à 10.

**17.** Produit de diagnostic de la toxoplasmose, y compris de la toxoplasmose congénitale, caractérisé en ce qu'il contient en tant que constituant actif, une préparation antigénique ou des antigènes selon l'une quelconque des revendications 1 à 10.

**18.** Produit de diagnostic selon la revendication 17, caractérisé en ce qu'il comprend l'antigène de poids moléculaire 105 kDa, qui correspond à un marqueur de la phase chronique de la toxoplasmose.

**19.** Produit de diagnostic selon la revendication 17, caractérisé en ce qu'il comprend l'antigène de poids moléculaire 84 kDa, qui correspond à un marqueur transitoire de la phase chronique de la toxoplasmose.

**20.** Produit de diagnostic selon la revendication 17, caractérisé en ce qu'il comprend l'antigène de poids moléculaire 98 kDa, qui correspond à un marqueur de la phase aiguë de la toxoplasmose.

**21.** Produit de diagnostic selon la revendication 17, caractérisé en ce qu'il comprend l'antigène de poids moléculaire 68 kDa, qui correspond à un marqueur de la phase aiguë.

**22.** Produit de diagnostic selon la revendication 17, caractérisé en ce qu'il comprend l'antigène de poids moléculaire 24 kDa, qui correspond à un marqueur tardif de la phase chronique de la toxoplasmose ainsi qu'à un marqueur d'immunité protectrice.

**23.** Procédé de détection et/ou de dépistage de la toxoplasmose, caractérisé en ce qu'il comprend la mise en contact d'un sérum humain avec un produit de diagnostic selon l'une quelconque des revendications 17 à 22.

**Revendications pour les Etats contractants suivants : ES, GR**

**1.** Procédé d'obtention d'une préparation antigénique de *Toxoplasma gondii* (TG) contenant un mélange d'antigènes d'excrétion-sécrétion de tachyzoïtes de TG, capables d'induire une réponse humorale protectrice de type IgE contre la toxoplasmose, caractérisé en ce qu'il comprend les étapes suivantes :
- incubation d'une suspension de tachyzoïtes de *Toxoplasma gondii* pendant environ 3 heures dans un milieu acellulaire de type RPMI 1640 contenant 10 % de sérum décomplémenté d'origine humaine ou animale ;
- centrifugation de la suspension incubée et récupération des surnageants dépourvus de corps parasitaires et contenant les antigènes d'excrétion-sécrétion (antigènes ES) ;

- filtration desdits surnageants, pour l'obtention de ladite préparation antigénique ; et éventuellement

- concentration et congélation à - 70°C de ladite préparation antigénique.

2. Procédé selon la revendication 1, caractérisé en ce que la préparation antigénique obtenue est constituée par des antigènes d'excrétion-sécrétion de tachyzoïtes de TG, de poids moléculaires respectifs 185 kDa, 170 kDa, 155 kDa, 105 kDa, 98 kDa, 84 kDa, 68 kDa, 57 kDa, 53 kDa, 45 kDa, 43 kDa, 39 kDa, 35 kDa, 34 kDa, 31 kDa, 30 kDa, 26 kDa, 25 kDa, 24 kDa et 20 kDa.

3. Procédé de préparation d'antigènes ES de *Toxoplasma gondii* à partir de la préparation antigénique préparée selon la revendication 1 ou la revendication 2, caractérisé en ce qu'il comprend les étapes suivantes :
- immunoprécipitation des différents antigènes ES contenus dans ladite préparation, préalablement radiomarqués, par mise en contact de cette dernière avec un sérum de mammifère, notamment sérum humain ou animal convenable choisi dans le groupe qui comprend les sérums prélevés chez des malades et reconnus comme séropositifs (soit de phase aiguë contenant des IgM spécifiques, soit de phase subaiguë contenant à la fois des IgM et des IgG, soit de phase chronique contenant des IgG) et les sérums obtenus par immunisation par une préparation antigénique selon la revendication 1 ou la revendication 2 et contenant des anticorps anti-ES ;
- piégeage des complexes antigènes-anticorps formés (immunoprécipités) sur des billes de protéine A-Sépharose®, éventuellement traitées de manière à fixer les anticorps de type IgG et les anticorps de type IgM ;
- séparation desdits antigènes ES par centrifugation et récupération des surnageants contenant les immunoprécipités dissociés, et éventuellement
- électrophorèse des immunoprécipités en gels de polyacrylamide.

4. Procédé de préparation selon la revendication 3, caractérisé en ce que les antigènes ES obtenus présentent un poids moléculaire 185 kDa, 170 kDa, 155 kDa, 105 kDa, 57 kDa, 53 kDa, 45 kDa, 43 kDa, 39 kDa, 35 kDa, 34 kDa, 31 kDa, 30 kDa, 26 kDa, 25 kDa et 24 kDa et sont reconnus au cours de la phase chronique.

5. Procédé de préparation selon la revendication 3, caractérisé en ce que l'antigène ES obtenu présente un poids moléculaire de 105 kDa et correspond à un marqueur de la phase chronique de la toxoplasmose.

6. Procédé de préparation selon la revendication 3, caractérisé en ce que l'antigène ES obtenu présente un poids moléculaire de 84 kDa et correspond à un marqueur transitoire de la phase chronique de la toxoplasmose.

7. Procédé de préparation selon la revendication 3, caractérisé en ce que l'antigène ES obtenu présente un poids moléculaire de 98 kDa et correspond à un marqueur de la phase aiguë de la toxoplasmose.

8. Procédé de préparation selon la revendication 3, caractérisé en ce que l'antigène ES obtenu présente un poids moléculaire de 68 kDa et correspond à un marqueur de la phase aiguë de la toxoplasmose.

9. Procédé de préparation d'antigènes ES communs aux tachyzoïtes et aux bradyzoïtes de TG, de poids moléculaire d'environ 25 kDa-24 kDa, à partir de la préparation antigénique préparée selon la revendication 1 ou la revendication 2, caractérisé en ce qu'il comprend à la fois l'immunoprécipitation de ladite préparation antigénique :
- avec un sérum convenable choisi dans le groupe qui comprend les sérums prélevés chez des malades et les sérums obtenus par immunisation par une préparation antigénique selon la revendication 1 ou la revendication 2 et contenant des anticorps anti-ES et
- avec des sérums obtenus par immunisation par des antigènes de bradyzoïtes.

10. Procédé de préparation d'antigènes ES communs aux tachyzoïtes et aux bradyzoïtes de TG, de poids moléculaire d'environ 43 kDa et de nature glycoprotéique, à partir de la préparation antigénique préparée selon la revendication 1 ou la revendication 2, caractérisé en ce qu'il comprend à la fois l'immunoprécipitation de ladite préparation antigénique :

- avec un sérum convenable choisi dans le groupe qui comprend les sérums prélevés chez des malades et les sérums obtenus par immunisation par une préparation antigénique selon la revendication 1 ou la revendication 2 et contenant des anticorps anti-ES et
- avec des sérums obtenus par immunisation par des antigènes de bradyzoïtes.

11. Procédé de préparation d'un produit de traduction d'ARNm de tachyzoïtes, comprenant au moins un antigène ES selon l'une quelconque des revendications 3 à 10 et reconnu par des sérums humains de phase chronique ou de phase aiguë de l'infection à *Toxoplasma gondii,* caractérisé en ce qu'il comprend la traduction *in vitro* des ARNm, extraits de lysats de tachyzoïtes, dans du lysat de réticulocytes, en présence d'un mélange d'aminoacides dépourvus de L-méthionine et comprenant la $^{35}$S-méthionine, en milieu tamponné.

12. Procédé de clonage des produits de traduction selon la revendication 11, caractérisé en ce qu'il comprend :
   - l'extraction d'ARNm à partir de lysats de tachyzoïtes,
   - et la synthèse d'ADNc à partir desdits ARNm de tachyzoïtes précédemment obtenus, par action de la transcriptase réverse d'AMV, la banque d'ADNc ainsi formée étant ensuite criblée pour identifier les phages recombinants dont l'insertion d'ADNc dirige la synthèse d'une protéine ou fraction de protéine portant les épitopes reconnus par les anticorps anti-ES et y sélectionner les clones ES reconnus par des sérums humains, les clones retenus étant caractérisés par sélection d'anticorps.

13. Procédé de préparation d'un sérum riche en anticorps IgE propres à conférer une protection significative contre la toxoplasmose et en particulier contre les tachyzoïtes, caractérisé en ce qu'il comprend l'immunisation d'animaux par des préparations antigéniques, des antigènes ES ou des produits de traduction d'ARNm de tachyzoïtes selon l'une quelconque des revendications 1 à 11 et l'isolement dudit sérum.

14. Procédé de préparation d'un anticorps monoclonal, caractérisé en ce qu'il comprend la production des hybridomes résultant de la fusion de cellules d'animaux immunisés par les antigènes ES selon l'une quelconque des revendications 3 à 10 avec des cellules tumorales.

15. Procédé de préparation d'un vaccin propre à procurer une protection significative contre la toxoplasmose, caractérisé en ce que ledit vaccin est obtenu à partir d'une préparation antigénique ou un antigène ES selon l'une quelconque des revendications 1 à 10.

16. Procédé de préparation d'un produit de diagnostic de la toxoplasmose, y compris de la toxoplasmose congénitale, caractérisé en ce que ledit produit de diagnostic est obtenu à partir d'une préparation antigénique ou des antigènes selon l'une quelconque des revendications 1 à 10.

17. Procédé de préparation selon la revendication 16, caractérisé en ce que l'antigène a un poids moléculaire de 105 kDa, qui correspond à un marqueur de la phase chronique de la toxoplasmose.

18. Procédé de préparation selon la revendication 16, caractérisé en ce que l'antigène a un poids moléculaire de 84 kDa, qui correspond à un marqueur transitoire de la phase chronique de la toxoplasmose.

19. Procédé de préparation selon la revendication 16, caractérisé en ce que l'antigène a un de poids moléculaire de 98 kDa, qui correspond à un marqueur de la phase aiguë de la toxoplasmose.

20. Procédé de préparation selon la revendication 16, caractérisé en ce que l'antigène a un poids moléculaire de 68 kDa, qui correspond à un marqueur de la phase aiguë.

21. Procédé de préparation selon la revendication 16, caractérisé en ce que l'antigène a un poids moléculaire de 24 kDa, qui correspond à un marqueur tardif de la phase chronique de la toxoplasmose ainsi qu'à un marqueur d'immunité protectrice.

22. Procédé de détection et/ou de dépistage de la toxoplasmose, caractérisé en ce qu'il comprend la mise en contact d'un sérum humain avec un produit de diagnostic selon l'une quelconque des revendications 16 à 21.

**Claims**
**Claims for the following Contracting States : AT, BE, CH, DE, FR, GB, IT, LI, LU, NL, SE**

1. An antigenic preparation from *Toxoplasma gondii* (TG), characterized in that
   * it can be obtained by:
     - incubation of a suspension of *Toxoplasma gondii* tachyzoites for about 3 hours in a cell-free medium of the RPMI 1640 type containing 10% of decomplemented serum of human or animal origin;
     - centrifugation of the incubated suspension and recovery of the supernatants devoid of parasitic substances and containing the excretion-secretion antigens (ES antigens);
     - filtration of said supernatants to give said antigenic preparation; and optionally
     - concentration of said antigenic preparation and freezing at -70°C, and in that
   * it contains a mixture of excretion-secretion antigens from TG tachyzoites, capable of inducing an IgE-type protective humoral response against toxoplasmosis.

2. An antigenic preparation according to Claim 1, characterized in that it consists of excretion-secretion antigens from TG tachyzoites, having respective molecular weights of 185 kDa, 170 kDa, 155 kDa, 105 kDa, 98 kDa, 84 kDa, 68 kDa, 57 kDa, 53 kDa, 45 kDa, 43 kDa, 39 kDa, 35 kDa, 34 kDa, 31 kDa, 30 kDa, 26 kDa, 25 kDa, 24 kDa and 20 kDa.

3. ES antigens from *Toxoplasma gondii,* characterized in that they can be obtained from the antigenic preparation according to Claim 1 or Claim 2 by:
   - immunoprecipitation of the various ES antigens contained in said preparation, which have been radiolabelled beforehand, by bringing said preparation into contact with a mammalian serum, especially a suitable human or animal serum selected from the group comprising sera taken from patients and recognized as seropositive (either of the acute phase containing specific IgMs, or of the subacute phase containing both IgMs and IgGs, or of the chronic phase containing IgGs) and sera obtained by immunization with an antigenic preparation according to Claim 1 or Claim 2 and containing anti-ES antibodies;
   - trapping of the antigen/antibody complexes formed (immunoprecipitates) on beads of A-Sepharose® protein which have optionally been treated so as to bind the IgG-type antibodies and the IgM-type antibodies;
   - separation of said ES antigens by centrifugation and recovery of the supernatants containing the dissociated immunoprecipitates; and optionally
   - polyacrylamide gel electrophoresis of the immunoprecipitates.

4. ES antigens according to Claim 3, characterized in that they have a molecular weight of 185 kDa, 170 kDa, 155 kDa, 105 kDa, 57 kDa, 53 kDa, 45 kDa, 43 kDa, 39 kDa, 35 kDa, 34 kDa, 31 kDa, 30 kDa, 26 kDa, 25 kDa and 24 kDa and are recognized during the chronic phase.

5. An ES antigen according to Claim 3, characterized in that it has a molecular weight of 105 kDa and in that it corresponds to a marker of the chronic phase of toxoplasmosis.

6. An ES antigen according to Claim 3, characterized in that it has a molecular weight of 84 kDa and in that it corresponds to a transitory marker of the chronic phase of toxoplasmosis.

7. An ES antigen according to Claim 3, characterized in that it has a molecular weight of 98 kDa and in that it corresponds to a marker of the acute phase of toxoplasmosis.

8. An ES antigen according to Claim 3, characterized in that it has a molecular weight of 68 kDa and in that it corresponds to a marker of the acute phase of toxoplasmosis.

9. ES antigens according to Claim 3, characterized in that they can be obtained simultaneously by immunoprecipitation of the antigenic preparation according to Claim 1 or Claim 2:

- with a suitable serum selected from the group comprising sera taken from patients and sera obtained by immunization with an antigenic preparation according to Claim 1 or Claim 2 and containing anti-ES antibodies; and
- with sera obtained by immunization with antigens from bradyzoites, and

in that they correspond to ES antigens common to TG tachyzoites and bradyzoites, having a molecular weight of 25 kDa - 24 kDa.

10. ES antigens according to Claim 3, characterized in that they can be obtained simultaneously by immunoprecipitation of the antigen preparation according to Claim 1 or Claim 2:
- with a suitable serum selected from the group comprising sera taken from patients and sera obtained by immunization with an antigenic preparation according to Claim 1 or Claim 2 and containing anti-ES antibodies; and
- with sera obtained by immunization with antigens from bradyzoites, and

in that they correspond to ES antigens common to TG tachyzoites and bradyzoites, having a molecular weight of about 43 kDa and the nature of a glycoprotein.

11. A product of the translation of mRNAs from tachyzoites, characterized in that it comprises at least one ES antigen according to any one of Claims 3 to 10 and in that it is recognized by human sera of the chronic phase or acute phase of infection with *Toxoplasma gondii.*

12. A product of the translation of mRNAs from tachyzoites according to Claim 11, characterized in that it can be obtained by *in vitro* translation of mRNAs extracted from lyzates of tachyzoites, in reticulocyte lyzate, in the presence of a mixture of amino acids devoid of L-methionine and comprising $^{35}$S-methionine, in a buffered medium.

13. A method of cloning the translation products according to Claim 11 or Claim 12, characterized in that it comprises:
- the extraction of mRNAs from lyzates of tachyzoites; and
- the synthesis of cDNAs from said tachyzoite mRNAs obtained above, by reaction with reverse transcriptase from AMV, the cDNA library thus formed then being screened so as to identify the recombinant phages, the insertion of whose cDNAs directs the synthesis of a protein or protein fraction carrying the epitopes recognized by the anti-ES antibodies, and so as to select therefrom the ES clones recognized by human sera, the selected clones being characterized by antibody selection.

14. A serum rich in IgE antibodies suitable for conferring a significant protection against toxoplasmosis and in particular against the tachyzoites, characterized in that it can be obtained from animals immunized with antigenic preparations, ES antigens or products of the translation of mRNAs from tachyzoites according to any one of Claims 1 to 12.

15. A monoclonal antibody, characterized in that it can be produced by hybridomas resulting from the fusion of cells of animals immunized with the ES antigens according to any one of Claims 3 to 10, with tumoral cells.

16. A vaccine suitable for providing a significant protection against toxoplasmosis, characterized in that it contains, as the active constituent, an antigenic preparation or an ES antigen according to any one of Claims 1 to 10.

17. A product for the diagnosis of toxoplasmosis, including congenital toxoplasmosis, characterized in that it contains, as the active constituent, an antigenic preparation or antigens according to any one of Claims 1 to 10.

18. A diagnostic product according to Claim 17, characterized in that it comprises the antigen of molecular weight 105 kDa, which corresponds to a marker of the chronic phase of toxoplasmosis

19. A diagnostic product according to Claim 17, characterized in that it comprises the antigen of molecular weight 84 kDa, which corresponds to a transitory marker of the chronic phase of toxoplasmosis.

## EP 0 301 961 B1

**20.** A diagnostic product according to Claim 17, characterized in that it comprises the antigen of molecular weight 98 kDa, which corresponds to a marker of the acute phase of toxoplasmosis.

**21.** A diagnostic product according to Claim 17, characterized in that it comprises the antigen of molecular weight 68 kDa, which corresponds to a marker of the acute phase.

**22.** A diagnostic product according to Claim 17, characterized in that it comprises the antigen of molecular weight 24 kDa, which corresponds to a late marker of the chronic phase of toxoplasmosis and to a marker of protective immunity.

**23.** A method of detecting and/or screening toxoplasmosis, characterized in that it comprises bringing a human serum into contact with a diagnostic product according to any one of Claims 17 to 22.

**Claims for the following Contracting States : ES, GR**

**1.** A method of obtaining an antigenic preparation from *Toxoplasma gondii* (TG) containing a mixture of excretion-secretion antigens from TG tachyzoites, capable of inducing an IgE-type protective humoral response against toxoplasmosis, characterized in that it comprises the following steps:
   - incubation of a suspension of *Toxoplasma gondii* tachyzoites for about 3 hours in a cell-free medium of the RPMI 1640 type containing 10% of decomplemented serum of human or animal origin;
   - centrifugation of the incubated suspension and recovery of the supernatants devoid of parasitic substances and containing the excretion-secretion antigens (ES antigens);
   - filtration of said supernatants to give said antigenic preparation; and optionally
   - concentration of said antigenic preparation and freezing at -70° C.

**2.** A method according to Claim 1, characterized in that the antigenic preparation obtained consists of excretion-secretion antigens from TG tachyzoites, having respective molecular weights of 185 kDa, 170 kDa, 155 kDa, 105 kDa, 98 kDa, 84 kDa, 68 kDa, 57 kDa, 53 kDa, 45 kDa, 43 kDa, 39 kDa, 35 kDa, 34 kDa, 31 kDa, 30 kDa, 26 kDa, 25 kDa, 24 kDa and 20 kDa.

**3.** A method of preparing ES antigens from *Toxoplasma gondii* starting from the antigenic preparation obtained according to Claim 1 or Claim 2, characterized in that it comprises the following steps:
   - immunoprecipitation of the various ES antigens contained in said preparation, which have been radiolabelled beforehand, by bringing said preparation into contact with a mammalian serum, especially a suitable human or animal serum selected from the group comprising sera taken from patients and recognized as seropositive (either of the acute phase containing specific IgMs, or of the subacute phase containing both IgMs and IgGs, or of the chronic phase containing IgGs) and sera obtained by immunization with an antigenic preparation according to Claim 1 or Claim 2 and containing anti-ES antibodies;
   - trapping of the antigen/antibody complexes formed (immunoprecipitates) on beads of A-Sepharose® protein which have optionally been treated so as to bind the IgG-type antibodies and the IgM-type antibodies;
   - separation of said ES antigens by centrifugation and recovery of the supernatants containing the dissociated immunoprecipitates; and optionally
   - polyacrylamide gel electrophoresis of the immunoprecipitates.

**4.** A preparative method according to Claim 3, characterized in that the ES antigens obtained have a molecular weight of 185 kDa, 170 kDa, 155 kDa, 105 kDa, 57 kDa, 53 kDa, 45 kDa, 43 kDa, 39 kDa, 35 kDa, 34 kDa, 31 kDa, 30 kDa, 26 kDa, 25 kDa and 24 kDa and are recognized during the chronic phase.

**5.** A preparative method according to Claim 3, characterized in that the ES antigen obtained has a molecular weight of 105 kDa and corresponds to a marker of the chronic phase of toxoplasmosis.

**6.** A preparative method according to Claim 3, characterized in that the ES antigen obtained has a molecular weight of 84 kDa and corresponds to a transitory marker of the chronic phase of toxoplasmosis.

**7.** A preparative method according to Claim 3, characterized in that the ES antigen obtained has a molecular weight of 98 kDa and corresponds to a marker of the acute phase of toxoplasmosis.

**8.** A preparative method according to Claim 3, characterized in that the ES antigen obtained has a molecular weight of 68 kDa and corresponds to a marker of the acute phase of toxoplasmosis.

**9.** A method of preparing ES antigens common to TG tachyzoites and bradyzoites, having a molecular weight of about 25 kDa - 24 kDa, starting from the antigenic preparation obtained according to Claim 1 or Claim 2, characterized in that it comprises the simultaneous immunoprecipitation of said antigenic preparation:
- with a suitable serum selected from the group comprising sera taken from patients and sera obtained by immunization with an antigenic preparation according to Claim 1 or Claim 2 and containing anti-ES antibodies; and
- with sera obtained by immunization with antigens from bradyzoites.

**10.** A method of preparing ES antigens common to TG tachyzoites and bradyzoites, having a molecular weight of about 43 kDa and the nature of a glycoprotein, starting from the antigenic preparation obtained according to Claim 1 or Claim 2, characterized in that it comprises the simultaneous immunoprecipitation of said antigenic preparation:
- with a suitable serum selected from the group comprising sera taken from patients and sera obtained by immunization with an antigenic preparation according to Claim 1 or Claim 2 and containing anti-ES antibodies; and
- with sera obtained by immunization with antigens from bradyzoites.

**11.** A method of preparing a product of the translation of mRNAs from tachyzoites, comprising at least one ES antigen according to any one of Claims 3 to 10 and recognized by human sera of the chronic phase or acute phase of infection with *Toxoplasma gondii,* characterized in that it comprises *in vitro* translation of mRNAs extracted from lyzates of tachyzoites, in reticulocyte lyzate, in the presence of a mixture of amino acids devoid of L-methionine and comprising $^{35}$S-methionine, in a buffered medium.

**12.** A method of cloning the translation products according to Claim 11, characterized in that it comprises:
- the extraction of mRNAs from lyzates of tachyzoites; and
- the synthesis of cDNAs from said tachyzoite mRNAs obtained above, by reaction with reverse transcriptase from AMV, the cDNA library thus formed then being screened so as to identify the recombinant phages, the insertion of whose cDNAs directs the synthesis of a protein or protein fraction carrying the epitopes recognized by the anti-ES antibodies, and so as to select therefrom the ES clones recognized by human sera, the selected clones being characterized by antibody selection.

**13.** A method of preparing a serum rich in IgE antibodies suitable for conferring a significant protection against toxoplasmosis and in particular against the tachyzoites, characterized in that it comprises immunizing animals with antigenic preparations, ES antigens or products of the translation of mRNAs from tachyzoites according to any one of Claims 1 to 11, and isolating said serum.

**14.** A method of preparing a monoclonal antibody, characterized in that it comprises producing hybridomas resulting from the fusion of cells of animals immunized with the ES antigens according to any one of Claims 3 to 10, with tumoral cells.

**15.** A method of preparing a vaccine suitable for providing a significant protection against toxoplasmosis, characterized in that said vaccine is obtained from an antigenic preparation or an ES antigen according to any one of Claims 1 to 10.

**16.** A method of preparing a product for the diagnosis of toxoplasmosis, including congenital toxoplasmosis, characterized in that said diagnostic product is obtained from an antigenic preparation or antigens according to any one of Claims 1 to 10.

**17.** A preparative method according to Claim 16, characterized in that the antigen has a molecular weight of 105 kDa, which corresponds to a marker of the chronic phase of toxoplasmosis.

**18.** A preparative method according to Claim 16, characterized in that the antigen has a molecular weight of 84 kDa, which corresponds to a transitory marker of the chronic phase of toxoplasmosis.

**19.** A preparative method according to Claim 16, characterized in that the antigen has a molecular weight of 98 kDa, which corresponds to a marker of the acute phase of toxoplasmosis.

**20.** A preparative method according to Claim 16, characterized in that the antigen has a molecular weight of 68 kDa, which corresponds to a marker of the acute phase.

**21.** A preparative method according to Claim 16, characterized in that the antigen has a molecular weight of 24 kDa, which corresponds to a late marker of the chronic phase of toxoplasmosis and to a marker of protective immunity.

**22.** A method of detecting and/or screening toxoplasmosis, characterized in that it comprises bringing a human serum into contact with a diagnostic product according to any one of Claims 16 to 21.

**Patentansprüche**
**Patentansprüche für folgende Vertragsstaaten : AT, BE, CH, DE, FR, IT, LI, LU, NL, SE**

**1.** Antigenes Toxoplasma gondii (TG)-Präparat, dadurch **gekennzeichnet,** daß es erhalten werden kann durch
   - etwa dreistündige Inkubation einer Suspension aus Tachyzoiten von Toxoplasma gondii in einem azellulären Medium vom Typ RPMI 1640, das 10% dekomplementiertes Serum menschlichen oder tierischen Ursprungs enthält;
   - Zentrifugation der inkubierten Suspension und Gewinnung der von Parasitenkörpern befreiten Überstände, die die Exkretions-Sekretionsantigene (ES-Antigene) enthalten;
   - Filtration der Überstände, um das antigene Präparat zu erhalten; und gegebenenfalls
   - Konzentrieren und Einfrieren auf -70°C des antigenen Präparats,
   und daß es ein Gemisch aus Exkretions-Sekretionsantigenen von TG-Tachyzoiten, mit der Fähigkeit eine schützende humorale Antwort des Typs IgE gegen Toxoplasmose zu induzieren, enthält.

**2.** Antigenes Präparat nach Anspruch 1, dadurch **gekennzeichnet,** daß es Exkretions-Sekretionsantigene von TG-Tachyzoiten mit jeweiligen Molekulargewichten von 185 kDa, 170 kDa, 155 kDa, 105 kDa, 98 kDa, 84 kDa, 68 kDa, 57 kDa, 53 kDa, 45 kDa, 43 kDa, 39 kDa, 35 kDa, 34 kDa, 31 kDa, 30 kDa, 26 kDa, 25 kDa, 24 kDa und 20 kDa enthält.

**3.** ES-Antigene von Toxoplasma gondii, dadurch **gekennzeichnet,** daß sie aus dem antigenen Präparat nach Anspruch 1 oder 2 durch
   - Immunpräzipitation der verschiedenen, in dem Präparat enthaltenen und zuvor radioaktiv markierten ES-Antigene durch Inkontaktbringen der letzteren mit einem Säugerserum, insbesondere einem menschlichen oder tierischen Serum, das geeigneterweise aus der Gruppe, umfassend Seren, die zuvor bei Kranken entnommen wurden und die als seropositiv bekannt sind (entweder aus der akuten Phase mit spezifischem IgM oder aus der subakuten Phase mit gleichzeitigem Vorhandensein von IgM und IgG oder aus der chronischen Phase mit IgG), und durch Immunisierung durch ein antigenes Präparat nach Anspruch 1 oder 2 erhaltene Seren, die Anti-ES-Antikörper enthalten;
   - Einfangen der gebildeten Antigen-Antikörperkomplexe (Immunpräzipitate) auf Protein A-Sepharose®-Kugeln, die gegebenenfalls so behandelt wurden, daß sie die Antikörper vom Typ IgG und die Antikörper vom Typ IgM fixieren;
   - Abtrennen der ES-Antigene durch Zentrifugation und Gewinnung der Überstände, die die dissoziierten Immunpräzipitate enthalten; und gegebenenfalls
   - Elektrophorese der Immunpräzipitate auf Polyacrylamidgelen
   erhalten werden können.

**4.** ES-Antigene nach Anspruch 3, dadurch **gekennzeichnet,** daß sie ein Molekulargewicht von 185 kDa, 170 kDa, 155 kDa, 105 kDa, 57 kDa, 53 kDa, 45 kDa, 43 kDa, 39 kDA, 35 kDa, 34 kDa, 31 kDa, 30 kDa, 26 kDa, 25 kDa und 24 kDa besitzen und im Verlauf der chronischen Phase erkannt werden.

**5.** ES-Antigen nach Anspruch 3, dadurch **gekennzeichnet,** daß es ein Molekulargewicht von 105 kDa besitzt und daß es einem Marker der chronischen Phase der Toxoplasmose entspricht.

**6.** ES-Antigen nach Anspruch 3, dadurch **gekennzeichnet,** daß es ein Molekulargewicht von 84 kDa besitzt und daß es einem vorübergehenden Marker der chronischen Phase der Toxoplasmose entspricht.

**7.** ES-Antigen nach Anspruch 3, dadurch **gekennzeichnet,** daß es ein Molekulargewicht von 98 kDa besitzt und daß es einem Marker der akuten Phase der Toxoplasmose entspricht.

**8.** ES-Antigen nach Anspruch 3, dadurch **gekennzeichnet,** daß es ein Molekulargewicht von 68 kDa besitzt und daß es einem Marker der akuten Phase der Toxoplasmose entspricht.

**9.** ES-Antigene nach Anspruch 3, dadurch **gekennzeichnet,** daß sie gleichzeitig durch Immunpräzipitation des antigenen Präparats nach Anspruch 1 oder 2
- mit einem geeigneten Serum, ausgewählt aus der Gruppe, umfassend zuvor bei Kranken entnommene Seren und durch Immunisierung mit einem antigenen Präparat nach Anspruch 1 oder 2 erhaltene Seren, die Anti-ES-Antikörper enthalten, und
- mit Seren, die durch Immunisieren mit Bradyzoiten-Antigenen erhalten wurden, und

daß sie den ES-Antigenen, die den Tachyzoiten und Bradyzoiten von TG gemeinsam sind und ein Molekulargewicht von etwa 25 kDa bis 24 kDa besitzen, entsprechen, erhalten werden können.

**10.** ES-Antigene nach Anspruch 3, dadurch **gekennzeichnet,** daß sie gleichzeitig durch Immunpräzipitation des antigenen Präparats nach Anspruch 1 oder 2
- mit einem geeigneten Serum, ausgewählt aus der Gruppe, umfassend zuvor bei Kranken entnommene Seren und durch Immunisieren mit einem antigenen Präparat nach Anspruch 1 oder 2 erhaltene Seren, die Anti-ES-Antikörper enthalten; und
- mit durch Immunisieren mit Antigenen der Bradyzoiten erhaltenen Seren erhalten werden können, und

daß sie den ES-Antigenen, die den Tachyzoiten und den Bradyzoiten von TG gemeinsam sind, ein Molekulargewicht von etwa 43 kDa besitzen und glycoproteinischer Natur sind, entsprechen.

**11.** Translationsprodukt der mRNA von Tachyzoiten, dadurch **gekennzeichnet,** daß es mindestens ein ES-Antigen nach einem der Ansprüche 3 bis 10 umfaßt, und daß es von menschlichen Seren der chronischen Phase oder der akuten Phase der Infektion mit Toxoplasma gondii erkannt wird.

**12.** Translationsprodukt der mRNA von Tachyzoiten nach Anspruch 11, dadurch **gekennzeichnet,** daß es durch in vitro-Translation der mRNA, die aus Tachyzoitenlysaten extrahiert wurde, in einem Reticulocytenlysat in Gegenwart eines Gemisches aus Aminosäuren ohne L-Methionin und mit $^{35}$ S-Methionin in einem Puffermedium erhalten werden kann.

**13.** Verfahren zur Clonierung der Translationsprodukte nach Anspruch 11 oder 12, dadurch **gekennzeichnet,** daß man
- die mRNA aus Tachyzoitenlysaten extrahiert;
- cDNA aus den zuvor erhaltenen mRNAs von Tachyzoiten durch die reverse AMV-Transkriptase synthetisiert, die so gebildete cDNA-Bank anschließend absucht, um die rekombinanten Phagen zu identifizieren, deren cDNA Insertion die Synthese eines Proteins oder einer Proteinfraktion dirigiert, das bzw. die die Epitope trägt, die durch Anti-ES-Antikörper erkannt werden, und daraus die durch die menschlichen Seren erkannten ES-Clone selektioniert, wobei die erhaltenen Clone durch die Antikörper-Selektion charakterisiert sind.

**14.** An IgE-Antikörpern reiches Serum, das geeignet ist einen signifikanten Schutz gegen die Toxoplasmose und insbesondere gegen die Tachyzoiten zu verleihen, dadurch **gekennzeichnet,** daß es aus mit antigenen Präparaten der ES-Antigene oder mit Translationsprodukten der mRNA von Tachyzoiten nach einem der Ansprüche 1 bis 12 immunisierten Tieren erhalten werden kann.

**15.** Monoclonaler Antikörper, dadurch **gekennzeichnet,** daß er durch Hybridome aus der Fusion von Zellen von Tieren, die mit den ES-Antigenen nach einem der Ansprüche 3 bis 10 immunisiert worden sind, mit Tumorzellen erzeugt werden kann.

**16.** Impfstoff, geeignet einen signifikanten Schutz gegen Toxoplasmose zu verleihen, dadurch **gekennzeichnet,** daß er als Wirkstoff ein antigenes Präparat oder ein ES-Antigen nach einem der Ansprüche 1 bis 10 enthält.

**17.** Produkt zur Diagnose der Toxoplasmose einschließlich der congenitalen Toxoplasmose, dadurch **gekennzeichnet,** daß es als Wirkstoff ein antigenes Präparat oder Antigene nach einem der Ansprüche 1 bis 10 enthält.

**18.** Produkt zur Diagnose nach Anspruch 17, dadurch **gekennzeichnet,** daß es das Antigen mit einem Molekulargewicht von 105 kDa, das einem Marker der chronischen Phase der Toxoplasmose entspricht, umfaßt.

**19.** Produkt zur Diagnose nach Anspruch 17, dadurch **gekennzeichnet,** daß es das Antigen mit einem Molekulargewicht von 84 kDa, das einem vorübergehenden Marker der chronischen Phase der Toxoplasmose entspricht, umfaßt.

**20.** Produkt zur Diagnose nach Anspruch 17, dadurch **gekennzeichnet,** daß es das Antigen mit einem Molekulargewicht von 98 kDa, das einem Marker der akuten Phase der Toxoplasmose entspricht, umfaßt.

**21.** Produkt zur Diagnose nach Anspruch 17, dadurch **gekennzeichnet,** daß es das Antigen mit einem Molekulargewicht von 68 kDa, das einem Marker der akuten Phase entspricht, umfaßt.

**22.** Produkt zur Diagnose nach Anspruch 17, dadurch **gekennzeichnet,** daß es das Antigen mit einem Molekulargewicht von 24 kDa, das einem späten Marker der chronischen Phase der Toxoplasmose sowie einem Marker der Schutzimmunität entspricht, umfaßt.

**23.** Verfahren zum Nachweis und/oder zum Aufspüren der Toxoplasmose, dadurch **gekennzeichnet,** daß man ein menschliches Serum mit einem Produkt zur Diagnose nach einem der Ansprüche 17 bis 22 in Kontakt bringt.

**Patentansprüche für folgende Vertragsstaaten : ES, GR**

**1.** Verfahren zur Gewinnung eines antigenen Toxoplasma gondii (TG)-Präparats, das ein Gemisch aus Exkretions-Sekretionsantigenen von TG-Tachyzoiten enthält, welches eine schützende humorale Antwort vom Typ IgE gegen Toxoplasmose induzieren kann, dadurch **gekennzeichnet,** daß es die folgenden Stufen umfaßt:
   - etwa dreistündige Inkubation einer Suspension aus Tachyzoiten von Toxoplasma gondii in einem azellulären Medium vom Typ RPMI 1640, das 10% dekomplementiertes Serum menschlichen oder tierischen Ursprungs enthält;
   - Zentrifugation der inkubierten Suspension und Gewinnung der von Parasitenkörpern befreiten Überstände, die die Exkretions-Sekretionsantigene (ES-Antigene) enthalten;
   - Filtration der Überstände, um das antigene Präparat zu erhalten; und gegebenenfalls
   - Konzentrieren und Einfrieren auf -70 °C des antigenen Präparats.

**2.** Verfahren nach Anspruch 1, dadurch **gekennzeichnet,** daß das erhaltene antigene Präparat Exkretions-Sekretionsantigene von TG-Tachyzoiten mit jeweiligen Molekulargewichten von 185 kDa, 170 kDa, 155 kDa, 105 kDa, 98 kDa, 84 kDa, 68 kDa, 57 kDa, 53 kDa, 45 kDa, 43 kDa, 39 kDa, 35 kDa, 34 kDa, 31 kDa, 30 kDa, 26 kDa, 25 kDa, 24 kDa und 20 kDa enthält.

**3.** Verfahren zur Herstellung von ES-Antigenen von Toxoplasma gondii aus dem nach Anspruch 1 oder 2 hergestellten antigenen Präparat, dadurch **gekennzeichnet,** daß es die folgenden Stufen umfaßt:
   - Immunpräzipitation der verschiedenen, in dem Präparat enthaltenen und zuvor radioaktiv markierten ES-Antigene durch Inkontaktbringen der letzteren mit einem Säugerserum, insbesondere einem menschlichen oder tierischen Serum, das geeigneterweise aus der Gruppe, umfassend Seren, die zuvor bei Kranken entnommen wurden und die als seropositiv bekannt sind (entweder aus der akuten Phase mit spezifischem IgM oder aus der subakuten Phase mit gleichzeitigem Vorhandensein von IgM und IgG oder aus der chronischen Phase mit IgG), und durch Immunisierung durch ein antigenes Präparat nach Anspruch 1 oder 2 erhaltene Seren, die Anti-ES-Antikörper enthalten;

- Einfangen der gebildeten Antigen-Antikörperkomplexe (Immunpräzipitate) auf Protein A-Sepharose®-Kugeln, die gegebenenfalls so behandelt wurden, daß sie die Antikörper vom Typ IgG und die Antikörper vom Typ IgM fixieren;

- Abtrennen der ES-Antigene durch Zentrifugation und Gewinnung der Überstände, die die dissoziierten Immunpräzipitate enthalten; und gegebenenfalls

- Elektrophorese der Immunpräzipitate auf Polyacrylamidgelen.

4. Verfahren zur Herstellung nach Anspruch 3, dadurch **gekennzeichnet,** daß die erhaltenen ES-Antigene ein Molekulargewicht von 185 kDa, 170 kDa, 155 kDa, 105 kDa, 57 kDa, 53 kDa, 45 kDa, 43 kDa, 39 kDa, 35 kDa, 34 kDa, 31 kDa, 30 kDa, 26 kDa, 25 kDa und 24 kDa besitzen und während der chronischen Phase erkannt werden.

5. Verfahren zur Herstellung nach Anspruch 3, dadurch **gekennzeichnet,** daß das erhaltene ES-Antigen ein Molekulargewicht von 105 kDa besitzt und einem Marker der chronischen Phase der Toxoplasmose entspricht.

6. Verfahren zur Herstellung nach Anspruch 3, dadurch **gekennzeichnet,** daß das erhaltene ES-Antigen ein Molekulargewicht von 84 kDa besitzt und einem vorübergehenden Marker der chronischen Phase der Toxoplasmose entspricht.

7. Verfahren zur Herstellung nach Anspruch 3, dadurch **gekennzeichnet,** daß das erhaltene ES-Antigen ein Molekulargewicht von 98 kDa besitzt und einem Marker der akuten Phase der Toxoplasmose entspricht.

8. Verfahren zur Herstellung nach Anspruch 3, dadurch **gekennzeichnet,** daß das erhaltene ES-Antigen ein Molekulargewicht von 68 kDa besitzt und einem Marker der akuten Phase der Toxoplasmose entspricht.

9. Verfahren zur Herstellung von ES-Antigenen, die Tachyzoiten und Bradyzoiten von TG gemeinsam sind und ein Molekulargewicht von etwa 25 kDa bis 24 kDa besitzen, aus einem nach Anspruch 1 oder 2 hergestellten antigenen Präparat, dadurch **gekennzeichnet,** daß man gleichzeitig das antigene Präparat

- mit einem geeigneten Serum, ausgewählt aus der Gruppe, umfassend zuvor bei Kranken entnommene Seren und durch Immunisierung mit einem antigenen Präparat nach Anspruch 1 oder 2 erhaltene Seren, die Anti-ES-Antikörper enthalten, und

- mit Seren, die durch Immunisieren mit Antigenen von Bradyzoiten erhalten wurden, immunpräzipitiert.

10. Verfahren zur Herstellung von ES-Antigenen, die Tachyzoiten und Bradyzoiten von TG gemeinsam sind, ein Molekulargewicht von etwa 43 kDa besitzen und glycoproteinischer Natur sind, aus dem nach Anspruch 1 oder 2 hergestellten antigenen Präparat, dadurch **gekennzeichnet,** daß man gleichzeitig das antigene Präparat

- mit einem geeigneten Serum, ausgewählt aus der Gruppe, umfassend die zuvor bei Kranken entnommenen Seren und die durch Immunisieren mit einem antigenen Präparat nach Anspruch 1 oder 2 erhaltenen Seren, die Anti-ES-Antikörper enthalten, und

- mit Seren, die durch Immunisieren mit Antigenen von Bradyzoiten erhalten wurden, immunpräzipitiert.

11. Verfahren zur Herstellung eines Translationsprodukts der mRNA von Tachyzoiten, umfassend mindestens ein ES-Antigen nach einem der Ansprüche 3 bis 10, welches von menschlichen Seren der chronischen Phase oder der akuten Phase der Toxoplasma gondii-Infektion erkannt wird, dadurch **gekennzeichnet,** daß man in vitro mRNA, extrahiert aus Tachyzoitenlysaten, in einem Reticulocytenlysat in Gegenwart eines Gemisches aus Aminosäuren ohne L-Methionin und mit $^{35}$S-Methionin in einem Puffermedium translatiert.

**12.** Verfahren zur Clonierung der Translationsprodukte nach Anspruch 10 oder 11, dadurch **gekennzeichnet,** daß man

- die mRNA aus Tachyzoitenlysaten extrahiert;
- cDNA aus den zuvor erhaltenen mRNAs von Tachyzoiten durch die reverse AMV-Transkriptase synthetisiert, die so gebildete cDNA-Bank anschließend absucht, um die rekombinanten Phagen zu identifizieren, deren cDNA-Insertion die Synthese eines Proteins oder einer Proteinfraktion dirigiert, das bzw. die die Epitope trägt, die durch Anti-ES-Antikörper erkannt werden, und daraus die durch die menschlichen Seren erkannten ES-Clone selektioniert, wobei die erhaltenen Clone durch die Antikörper-Selektion charakterisiert sind.

**13.** Verfahren zur Herstellung eines an IgE-Antikörpern reichen Serums, das einen signifikanten Schutz gegen Toxoplasmose und insbesondere gegen die Tachyzoiten verleihen kann, dadurch **gekennzeichnet,** daß man Tiere durch antigene Präparate, ES-Antigene oder Produkte der Translation der mRNA von Tachyzoiten nach einem der Ansprüche 1 bis 11 immunisiert und das Serum isoliert.

**14.** Verfahren zur Herstellung eines monoklonalen Antikörpers, dadurch **gekennzeichnet,** daß man Hybridome erzeugt, die aus der Fusion von Zellen von Tieren, die durch die ES-Antigene nach einem der Ansprüche 3 bis 10 immunisiert worden sind, mit Tumorzellen stammen.

**15.** Verfahren zur Herstellung eines Impfstoffs mit der Eignung einen signifikanten Schutz gegen Toxoplasmose zu verleihen, dadurch **gekennzeichnet,** daß der Impfstoff aus einem antigenen Präparat oder einem ES-Antigen nach einem der Ansprüche 1 bis 10 erhalten wurde.

**16.** Verfahren zur Herstellung eines Produkts zur Diagnose der Toxoplasmose einschließlich der congenitalen Toxoplasmose, dadurch **gekennzeichnet,** daß das diagnostische Produkt aus einem antigenen Präparat oder Antigenen nach einem der Ansprüche 1 bis 10 erhalten worden ist.

**17.** Verfahren zur Herstellung nach Anspruch 16, dadurch **gekennzeichnet,** daß das Antigen ein Molekulargewicht von 105 kDa besitzt, was einem Marker der chronischen Phase der Toxoplasmose entspricht.

**18.** Verfahren zur Herstellung nach Anspruch 16, dadurch **gekennzeichnet,** daß das Antigen ein Molekulargewicht von 84 kDa besitzt, was einem vorübergehenden Marker der chronischen Phase der Toxoplasmose entspricht.

**19.** Verfahren zur Herstellung nach Anspruch 16, dadurch **gekennzeichnet,** daß das Antigen ein Molekulargewicht von 98 kDa besitzt, was einem Marker der akuten Phase der Toxoplasmose entspricht.

**20.** Verfahren zur Herstellung nach Anspruch 16, dadurch **gekennzeichnet,** daß das Antigen ein Molekulargewicht von 68 kDa besitzt, was einem Marker der akuten Phase entspricht.

**21.** Verfahren zur Herstellung nach Anspruch 16, dadurch **gekennzeichnet,** daß das Antigen ein Molekulargewicht von 24 kDa besitzt, was einem späten Marker der chronischen Phase der Toxoplasmose sowie einem Marker der Schutzimmunität entspricht.

**22.** Verfahren zum Nachweis und/oder zum Aufspüren der Toxoplasmose, dadurch **gekennzeichnet,** daß man ein menschliches Serum mit einem diagnostischen Produkt nach einem der Ansprüche 16 bis 21 in Kontakt bringt.

FIG.1

FIG.2

FIG.3

FIG.4

FIG.5

# FIG.6

# FIG. 7

FIG. 8

# FIG. 9

# FIG. 10

# FIG. 11

FIG. 13

FIG. 12

# FIG. 14

# FIG.15